# EUROPÄISCHE PATENTSCHRIFT

(11) **EP 2 084 143 B1**
(45) Veröffentlichungstag und Bekanntmachung des Hinweises auf die Patenterteilung: **27.01.2010**
(21) Anmeldenummer: 07819435.4
(22) Anmeldetag: 30.10.2007
(51) Int. Cl.: C07D 337/08, A61K 31/08

(54) **NEUE MIT CYCLOHEXYLRESTEN SUBSTITUIERTE1,4-BENZOTHIEPIN-1,1 -DIOXIDDERIVATE, VERFAHREN ZU DEREN HERSTELLUNG, DIESE VERBINDUNGEN ENTHALTENDE ARZNEIMITTEL UND DEREN VERWENDUNG**
NOVEL 1,4-BENZOTHIEPIN-1,1 -DIOXIDE DERIVATIVES WHICH ARE SUBSTITUTED WITH CYCLOHEXYL GROUPS, METHOD FOR PRODUCING THE SAME, DRUGS CONTAINING SAID COMPOUNDS AND USE THEREOF
NOUVEAUX DÉRIVÉS DE 1,4-BENZOTHIÉPINE-1,1-DIOXYDE SUBSTITUÉS PAR DES RESTES CYCLOHEXYLE, PROCÉDÉ DE PRODUCTION APPROPRIÉ, MÉDICAMENTS CONTENANT LESDITS COMPOSÉS ET LEUR UTILISATION

(30) Priorität: 14.11.2006 DE 102006053636
(43) Veröffentlichungstag der Anmeldung: 05.08.2009
(73) Patentinhaber: Sanofi-Aventis Deutschland GmbH, 65929 Frankfurt am Main (DE)
(72) Erfinder: FRICK, Wendelin, 65926 Frankfurt am Main (DE); GLOMBIK, Heiner, 65926 Frankfurt am Main (DE); THEIS, Stefan, 65926 Frankfurt am Main (DE); HEUER, Hubert, 65926 Frankfurt am Main (DE); SCHAEFER, Hans-Ludwig, 65926 Frankfurt am Main (DE); KRAMER, Werner, 65926 Frankfurt am Main (DE)
(86) Internationale Anmeldenummer: PCT/EP2007/009394
(87) Internationale Veröffentlichungsnummer: WO 2008/058629

(56) Entgegenhaltungen:
- WO-A-03/018024
- WO-A-2004/076430
- US-A- 5 994 391

## Beschreibung

Neue mit Cyclohexylresten substituierte1,4-Benzothiepin-1,1-Dioxidderivate, Verfahren zu deren Herstellung, diese Verbindungen enthaltende Arzneimittel und deren Verwendung

Die Erfindung betrifft mit Cyclohexylresten substituierte 1,4-Benzothiepin-1,1-Dioxidderivate und deren physiologisch verträgliche Salze.

Es sind bereits strukturähnliche 1,4-Benzothiepin-1,1-Dioxidderivate beschrieben worden (US 5,994,391).

Der Erfindung lag die Aufgabe zugrunde, weitere Verbindungen zur Verfügung zu stellen, die eine hypolipidämische Wirkung zeigen.

Die Erfindung betrifft daher die Verbindung der Formel I 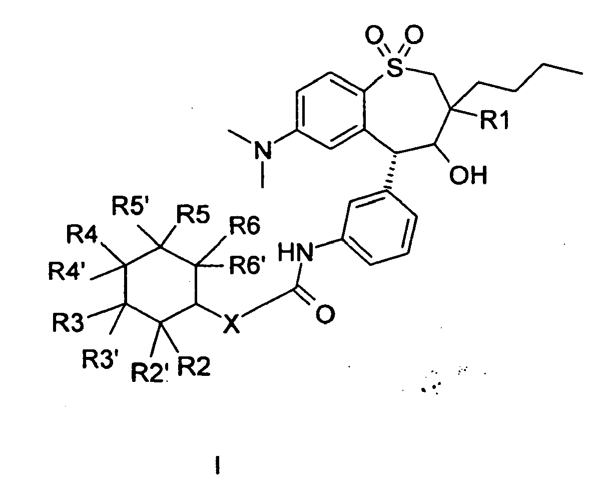 worin bedeuten
- X: O, NH;
- R1: (C₁-C₄)-Alkyl;
- R2,: R2', R3, R3', R4, R4', R5, R5', R6, R6' unabhängig voneinander H, OH, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; C(=NH)(NH₂), PO₃H₂, SF₅, SO₃H, OSO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, -(CH₂)-O-(CH₂)ₘ-Phenyl, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
sowie deren pharmazeutisch verträgliche Salze.
Bevorzugt sind Verbindungen der Formel I, worin bedeuten
- X: NH;
- R1: (C₁-C₄)-Alkyl;
- R2,: R2', R3, R3', R4, R4', R5, R5', R6, R6' unabhängig voneinander H, OH, F, Cl, Br,I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; C(=NH)(NH₂), PO₂H₂, SF₅, SO₃H, OSO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br,l, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
sowie deren pharmazeutisch verträgliche Salze.
Besonders bevorzugt sind Verbindungen der Formel I, worin bedeuten
- X: NH;
- R1: (C₁-C₄)-Alkyl;
- R2',: R3', R4', R5', R6' H;
- R2,: R3, R4, R5, R6 unabhängig voneinander OH, F, Cl, Br,I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; C(=NH)(NH₂), PO₃H₂, SF₅, SO₃H, OSO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br,l, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)2, SO₂-CH₃, COOH, COO-(C₁-C₆)-Alkyl, CONH₂;
sowie deren pharmazeutisch verträgliche Salze.

In einer Ausführungsform sind Verbindungen der Formel I bevorzugt, in denen der Rest X gleich O ist.

In einer Ausführungsform sind Verbindungen der Formel I bevorzugt, in denen der Rest X gleich NH ist.

In einer Ausführungsform sind Verbindungen der Formel I bevorzugt, in denen der Rest X gleich CH₂ ist.

In einer Ausführungsform sind Verbindungen der Formel I bevorzugt, in denen der Rest R1 gleich Buyl ist.

In einer Ausführungsform sind Verbindungen der Formel I bevorzugt, in denen der Rest R1 gleich Ethyl ist.

In einer Ausführungsform sind Verbindungen der Formel I bevorzugt, in denen der Rest zumindest einer der Reste R2, R2', R3, R3', R4, R4', R5, R5', R6 oder R6' gleich Benzyl ist.

In einer Ausführungsform sind Verbindungen der Formel I bevorzugt, in denen der Rest zumindest einer der Reste R2, R2', R3, R3', R4, R4', R5, R5', R6 oder R6' gleich OSO₃H ist.

Weiterhin bevorzugt sind Verbindungen der Formel I, worin ein, zwei oder drei der Reste R2, R2', R3, R3', R4, R4', R5, R5', R6, R6' die Bedeutung OH besitzen.

In einer Ausführungsform sind Verbindungen der Formel I bevorzugt, in denen die Struktur der Formel I wie folgt vorliegt:

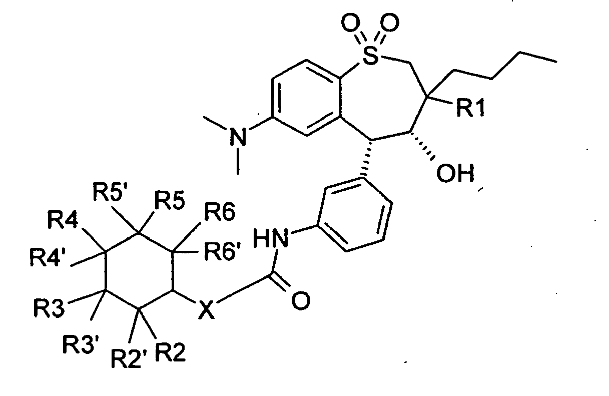

Können Reste oder Substituenten mehrfach in den Verbindungen der Formeln I auftreten, so können sie alle unabhängig voneinander die angegebene Bedeutung haben und gleich oder verschieden sein.

Die Alkyl-, Alkenyl-, Alkinyl-, Alkylen-, Alkenylen- und Alkinylenreste in den Resten R1, R2, R2', R3, R3', R4, R4', R5, R5', R6 und R6' können sowohl geradkettig wie verzweigt sein.

Die Erfindung bezieht sich auf Verbindungen der Formel I, in Form ihrer Tautomere, Racemate, racemischen Mischungen, Stereoisomerengemische, reinen Stereoisomere, Diastereoisomerengemische, reinen Diastereoisomere. Die Trennung der Gemische erfolgt zum Beispiel auf chromatographischem Weg.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze), Erdalkalisalze (wie Magnesium- und Calciumsalze), Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I wie vorstehend beschrieben, sowie ihre Salze und Solvate wie hierin beschrieben.

Unter einem Alkylrest wird eine geradkettige oder verzweigte Kohlenwasserstofflkette mit einem bis acht Kohlenstoffen verstanden, wie z.B. Methyl, Ethyl, iso-Propyl, tert.-Butyl, Hexyl, Heptyl, Octyl. Die Alkylreste können einfach oder mehrfach wie oben beschrieben substituiert sein.

Die Verbindung(en) der Formel I können auch in Kombination mit weiteren Wirkstoffen verabreicht werden.

Die Menge einer Verbindung gemäß Formel I, die erforderlich ist, um den gewünschten biologischen Effekt zu erreichen, ist abhängig von einer Reihe von Faktoren, z.B. der gewählten spezifischen Verbindung, der beabsichtigten Verwendung, der Art der Verabreichung und dem klinischen Zustand des Patienten. Im Allgemeinen liegt die Tagesdosis im Bereich von 0,3 mg bis 100 mg (typischerweise von 3 mg und 50 mg) pro Tag pro Kilogramm Körpergewicht, z.B. 3-10 mg/kg/Tag. Eine intravenöse Dosis kann z.B. im Bereich von 0,3 mg bis 1,0 mg/kg liegen, die geeigneterweise als Infusion von 10 ng bis 100 ng pro Kilogramm pro Minute verabreicht werden kann. Geeignete Infusionslösungen für diese Zwecke können z.B. von 0,1 ng bis 10 mg, typischerweise von 1 ng bis 10 mg pro Milliliter, enthalten. Einzeldosen können z.B. von 1 mg bis 10 g des Wirkstoffs enthalten. Somit können Ampullen für Injektionen beispielsweise von 1 mg bis 100 mg, und oral verabreichbare Einzeldosisformulierungen, wie zum Beispiel Tabletten oder Kapseln, können beispielsweise von 1,0 bis 1000 mg, typischerweise von 10 bis 600 mg enthalten. Zur Therapie der oben genannten Zustände können die Verbindungen gemäß Formel **I** selbst als Verbindung verwendet werden, vorzugsweise liegen sie jedoch mit einem verträglichen Träger in Form einer pharmazeutischen Zusammensetzung vor. Der Träger muss natürlich verträglich sein, in dem Sinne, dass er mit den anderen Bestandteilen der Zusammensetzung kompatibel ist und nicht gesundheitsschädlich für den Patienten ist. Der Träger kann ein Feststoff oder eine Flüssigkeit oder beides sein und wird vorzugsweise mit der Verbindung als Einzeldosis formuliert, beispielsweise als Tablette, die von 0,05% bis 95 Gew.-% des Wirkstoffs enthalten kann. Weitere pharmazeutisch aktive Substanzen können ebenfalls vorhanden sein, einschließlich weiterer Verbindungen gemäß Formel I. Die erfindungsgemäßen pharmazeutischen Zusammensetzungen können nach einer der bekannten pharmazeutischen Methoden hergestellt werden, die im wesentlichen darin bestehen, dass die Bestandteile mit pharmakologisch verträglichen Träger- und/oder Hilfsstoffen gemischt werden.

Erfindungsgemäße pharmazeutische Zusammensetzungen sind solche, die für orale, rektale, topische, perorale (z.B. sublinguale) und parenterale (z.B. subkutane, intramuskuläre, intradermale oder intravenöse) Verabreichung geeignet sind, wenngleich die geeignetste Verabreichungsweise in jedem Einzelfall von der Art und Schwere des zu behandelnden Zustandes und von der Art der jeweils verwendeten Verbindung gemäß Formel I abhängig ist. Auch dragierte Formulierungen und dragierte Retardformulierungen gehören in den Rahmen der Erfindung. Bevorzugt sind säure- und magensaftresistente Formulierungen. Geeignete magensaftresistente Beschichtungen umfassen Celluloseacetatphthalat, Poylvinylacetatphthalat, Hydroxypropylmethylcellulosephthalat und anionische Polymere von Methacrylsäure und Methacrylsäuremethylester.

Geeignete pharmazeutische Verbindungen für die orale Verabreichung können in separaten Einheiten vorliegen, wie zum Beispiel Kapseln, Oblatenkapseln, Lutschtabletten oder Tabletten, die jeweils eine bestimmte Menge der Verbindung gemäß Formel I enthalten; als Pulver oder Granulate; als Lösung oder Suspension in einer wässrigen oder nicht-wässrigen Flüssigkeit; oder als eine Öl-in-Wasser- oder Wasser-in-Öl-Emulsion. Diese Zusammensetzungen können, wie bereits erwährt, nach jeder geeigneten pharmazeutischen Methode zubereitet werden, die einen Schritt umfasst, bei dem der Wirkstoff und der Träger (der aus einem oder mehreren zusätzlichen Bestandteilen bestehen kann) in Kontakt gebracht werden. Im allgemeinen werden die Zusammensetzungen durch gleichmäßiges und homogenes Vermischen des Wirkstoffs mit einem flüssigen und/oder feinverteilten festen Träger hergestellt, wonach das Produkt, falls erforderlich, geformt wird. So kann beispielsweise eine Tablette hergestellt werden, indem ein Pulver oder Granulat der Verbindung verpresst oder geformt wird, gegebenenfalls mit einem oder mehreren zusätzlichen Bestandteilen. Gepresste Tabletten können durch tablettieren der Verbindung in frei fließender Form, wie beispielsweise einem Pulver oder Granulat, gegebenenfalls gemischt mit einem Bindemittel, Gleitmittel, inertem Verdünner und/oder einem (mehreren) oberflächenaktiven/dispergierenden Mittel in einer geeigneten Maschine hergestellt werden. Geformte Tabletten können durch Formen der pulverförmigen, mit einem inerten flüssigen Verdünnungsmittel befeuchteten Verbindung in einer geeigneten Maschine hergestellt werden.

Pharmazeutische Zusammensetzungen, die für eine perorale (sublinguale) Verabreichung geeignet sind, umfassen Lutschtabletten, die eine Verbindung gemäß Formel I mit einem Geschmacksstoff enthalten, üblicherweise Saccharose und Gummi arabicum oder Tragant, und Pastillen, die die Verbindung in einer inerten Basis wie Gelatine und Glycerin oder Saccharose und Gummi arabicum umfassen.

Geeignete pharmazeutische Zusammensetzungen für die parenterale Verabreichung umfassen vorzugsweise sterile wässrige Zubereitungen einer Verbindung gemäß Formel I, die vorzugsweise isotonisch mit dem Blut des vorgesehenen Empfängers sind. Diese Zubereitungen werden vorzugsweise intravenös verabreicht, wenngleich die Verabreichung auch subkutan, intramuskulär oder intradermal als Injektion erfolgen kann. Diese Zubereitungen können vorzugsweise hergestellt werden, indem die Verbindung mit Wasser gemischt wird und die erhaltene Lösung steril und mit dem Blut isotonisch gemacht wird. Injizierbare erfindungsgemäße Zusammensetzungen enthalten im Allgemeinen von 0,1 bis 5 Gew.-% der aktiven Verbindung.

Geeignete pharmazeutische Zusammensetzungen für die rektale Verabreichung liegen vorzugsweise als Einzeldosis-Zäpfchen vor. Diese können hergestellt werden, indem man eine Verbindung gemäß Formel I mit einem oder mehreren herkömmlichen festen Trägern, beispielsweise Kakaobutter, mischt und das entstehende Gemisch in Form bringt.

Geeignete pharmazeutische Zusammensetzungen für die topische Anwendung auf der Haut liegen vorzugsweise als Salbe, Creme, Lotion, Paste, Spray, Aerosol oder Öl vor. Als Träger können Vaseline, Lanolin, Polyethylenglykole, Alkohole und Kombinationen von zwei oder mehreren dieser Substanzen verwendet werden. Der Wirkstoff ist im Allgemeinen in einer Konzentration von 0,1 bis 15 Gew.-% der Zusammensetzung vorhanden, beispielsweise von 0,5 bis 2%.

Auch eine transdermale Verabreichung ist möglich. Geeignete pharmazeutische Zusammensetzungen für transdermale Anwendungen können als einzelne Pflaster vorliegen, die für einen langzeitigen engen Kontakt mit der Epidermis des Patienten geeignet sind. Solche Pflaster enthalten geeigneterweise den Wirkstoff in einer gegebenenfalls gepufferten wässrigen Lösung, gelöst und/oder dispergiert in einem Haftmittel oder dispergiert in einem Polymer. Eine geeignete Wirkstoff-Konzentration beträgt ca. 1 % bis 35%, vorzugsweise ca. 3% bis 15%. Als eine besondere Möglichkeit kann der Wirkstoff, wie beispielsweise in Pharmaceutical Research, 2(6): 318 (1986) beschrieben, durch Elektrotransport oder lontophorese freigesetzt werden.
Als weitere Wirkstoffe für die Kombinationspräparate sind geeignet:
Alle Antidiabetika, die in der Roten Liste 2006, Kapitel 12 genannt sind; alle Abmagerungsmittel/Appetitzügler, die in der Roten Liste 2006, Kapitel 1 genannt sind; alle Lipidsenker, die in der Roten Liste 2006, Kapitel 58 genannt sind. Sie können mit der erfindungsgemäßen Verbindung der Formel I insbesondere zur synergistischen Wirkungsverbesserung kombiniert werden. Die Verabreichung der Wirkstoffkombination kann entweder durch getrennte Gabe der Wirkstoffe an den Patienten oder in Form von Kombinationspräparaten, worin mehrere Wirkstoffe in einer pharmazeutischen Zubereitung vorliegen, erfolgen. Die meisten der nachfolgend aufgeführten Wirkstoffe sind in USP Dictionary of USAN and International Drug Names, US Pharmacopeia, Rockville 2001, offenbart.

Antidiabetika umfassen Insulin und Insulinderivate, wie z.B. Lantus^{®} (siehe www.lantus.com) oder HMR 1964 oder Levemir® (insulin detemir) oder solche, wie sie in W02005005477 (Novo Nordisk) beschrieben sind, schnell wirkende Insuline (siehe US 6,221,633), inhalierbare Insuline, wie z. B. Exubera^{®} oder orale Insuline, wie z. B. IN-105 (Nobex) oder Oral-lyn ™ (Generex Biotechnology), GLP-1-Derivate wie z.B. Exenatide, Liraglutide oder diejenigen die in WO 98/08871, WO2005027978, W02006037811, W0200603781 0 von Novo Nordisk A/S, in WO 01/04156 von Zealand oder in WO 00/34331 von Beaufour-Ipsen offenbart wurden, Pramlintide Acetat (Symlin; Amylin Pharmaceuticals), sowie oral wirksame hypoglykämische Wirkstoffe.

Die oral wirksamen hypoglykämischen Wirkstoffe umfassen vorzugsweise Sulfonylharnstoffe,
Biguanidine,
Meglitinide,
Oxadiazolidindione,
Thiazolidindione,
Glukosidase-Inhibitoren,
Hemmstoffe der Glykogenphosphorylase,
Glukagon-Antagonisten,
Glukokinaseaktivatoren,
Inhibitoren der Fructose-1,6-bisphosphatase
Modulatoren des Glukosetransporters-4 (GLUT4),
Inhibitoren der Glutamin-Fructose-6-Phosphat-Amidotransferase (GFAT),
GLP-1-Agonisten, Kaliumkanalöffner, wie z.B. diejenigen, die in WO 97/26265 und WO 99/03861 von Novo Nordisk A/S offenbart wurden oder die, die in W02006045799 beschrieben sind (Solvay),
Inhibitoren der Dipeptidylpeptidase-IV (DPP-IV),
Insulin-Sensitizer,
Inhibitoren von Leberenzymen, die an der Stimulation der Glukoneogenese und/oder Glykogenolyse beteiligt sind,
Modulatoren der Glukoseaufnahme, des Glukosetransports und der Glukoserückresorption,
Hemmstoffe der 11 ß-HSD1,
Inhibitoren der Protein-Tyrosin-Phosphatase-1 B (PTP1 B),
Modulatoren des natrium-abhängigen Glukosetransporters 1 oder 2 (SGLT1, SGLT2), den Fettstoffwechsel verändernde Verbindungen wie antihyperlipidämische Wirkstoffe und antilipidämische Wirkstoffe,
Verbindungen, die die Nahrungsmitteleinnahme verringern,
Verbindungen, die die Thermogenese erhöhen,
PPAR- und RXR-Modulatoren und
Wirkstoffe, die auf den ATP-abhängigen Kaliumkanal der Betazellen wirken.

Bei einer Ausführungsform der Erfindung wird die Verbindungen der Formel I in Kombination mit einem HMGCoA-Reduktase Inhibitor wie Simvastatin, Fluvastatin, Pravastatin, Lovastatin, Atorvastatin, Cerivastatin, Rosuvastatin, L-659699 verabreicht.

Bei einer Ausführungsform der Erfindung wird die Verbindung der Formel I in Kombination mit einem Cholesterinresorptionsinhibitor, wie z.B. Ezetimibe, Tiqueside, Pamaqueside, FM-VP4 (sitostanol/campesterol ascorbyl phosphat; Forbes Medi-Tech, W02005042692, W02005005453), MD-0727 (Microbia Inc., W02005021497, W02005021495) oder mit Verbindungen, wie in W02002066464 (Kotobuki Pharmaceutical Co. Ltd.) oder W02005044256 oder W02005062824 (Merck & Co.) oder WO2005061451 und W02005061452 (AstraZeneca AB) und W02006017257 (Phenomix) oder WO2005033100 (Lipideon Biotechnology AG) beschrieben, verabreicht.

Bei einer Ausführungsform der Erfindung wird die Verbindung der Formel I in Kombination mit Vytorin™, einer festen Kombination von Ezetimibe mit Simvastatin, verabreicht.
Bei einer Ausführungsform der Erfindung wird die Verbindung der Formel I in Kombination mit einer festen Kombination von Ezetimibe mit Fenofibrat verabreicht.

Bei einer weiteren Ausführungsform der Erfindung wird die Verbindung der Formel I in Kombination mit einer festen Kombination von Fenofibrat mit Rosuvastatin verabreicht.

Bei einer Ausführungsform der Erfindung wird die Verbindung der Formel I in Kombination mit ISIS-301012, einem Antisense-Oligonukleotid, welches in der Lage ist, das Apolipoprotein B Gen zu regulieren, verabreicht.

Bei einer Ausführungsform der Erfindung wird die Verbindung der Formel I in Kombination mit einem PPAR gamma Agonisten, wie z.B. Rosiglitazon, Pioglitazon, JTT-501, Gl 262570, R-483, CS-011 (Rivoglitazon) verabreicht.

Bei einer Ausführungsform der Erfindung wird die Verbindung der Formel I in Kombination mit Competact™, einer festen Kombination von Pioglitazon Hydrochlorid mit Metformin Hydrochlorid, verabreicht.

Bei einer Ausführungsform der Erfindung wird die Verbindung der Formel I in Kombination mit duetact™, einer festen Kombination von Pioglitazon Hydrochlorid mit Glimepirid, verabreicht.

Bei einer Ausführungsform der Erfindung wird die Verbindung der Formel I in Kombination mit Avandamet^{®}, einer festen Kombination von Rosiglitazon Maleat mit Metformin Hydrochlorid, verabreicht.
Bei einer Ausführungsform der Erfindung wird die Verbindung der Formel I in Kombination mit PPAR alpha Agonisten, wie z.B. GW9578, GW-590735, K-111, LY-674, KRP-101, DRF-10945 verabreicht.

Bei einer Ausführungsform der Erfindung wird die Verbindung der Formel I in Kombination mit einem gemischten PPAR alpha/gamma Agonisten, wie z.B. Naveglitazar, LY-510929, ONO-5129, E-3030, AVE 8042, AVE 8134, AVE 0847, oder wie in PCT/US 00/11833, PCT/US 00/11490, DE10142734.4 oder in J.P.Berger et al., TRENDS in Pharmacological Sciences 28(5), 244-251, 2005 beschrieben, verabreicht.

Bei einer Ausführungsform der Erfindung wird die Verbindung der Formel I in Kombination mit einem PPAR delta Agonisten, wie z.B. GW-501516 verabreicht.

Bei einer Ausführungsform wird die Verbindung der Formel I in Kombination mit Metaglidasen oder mit MBX-2044 oder anderen partiellen PPAR gamma Agonisten/Antagonisten verabreicht

Bei einer weiteren Ausführungsform der Erfindung wird die Verbindung der Formel I in Kombination mit einem Aktivator der AMP-aktivierten Proteinkinase (AMPK), wie z. B. A-769662 oder solchen Verbindungen wie sie in US20050038068 beschrieben sind, verabreicht.

Bei einer Ausführungsform der Erfindung wird die Verbindung der Formel I in Kombination mit einem Fibrat, wie z.B. Fenofibrat, Clofibrat, Bezafibrat, verabreicht.

Bei einer Ausführungsform der Erfindung wird die Verbindung der Formel I in Kombination mit einem MTP-Inhibitor, wie z.B. Implitapide , BMS-201038, R-103757 oder solchen wie in W02005085226, W02005121091, WO2006010423 beschrieben, verabreicht.

Bei einer Ausführungsform der Erfindung wird die Verbindung der Formel I in Kombination mit einem CETP-Inhibitor, wie z.B. Torcetrapib oder JTT-705 oder solchen wie sie in W02006002342, W02006010422, WO2006012093 beschrieben sind, verabreicht.

Bei einer Ausführungsform der Erfindung wird die Verbindung der Formel I in Kombination mit Gallensäureresorptionsinhibitor (siehe z.B. US 6,245,744, US 6,221,897 oder WO00/61568), wie z.B. HMR 1741 oder solchen wie in DE 10 2005 033099.1 und DE 10 2005 033100.9 beschrieben, verabreicht.

Bei einer Ausführungsform der Erfindung wird die Verbindung der Formel I in Kombination mit einem polymeren Gallensäureadsorber, wie z.B. Cholestyramin, Colesevelam, verabreicht.

Bei einer Ausführungsform der Erfindung wird die Verbindung der Formel I in Kombination mit einem LDL-Rezeptorinducer (siehe US 6,342,512), wie z.B. HMR1171, HMR1586, oder solchen wie in W02005097738 beschrieben, verabreicht.

Bei einer Ausführungsform wird die Verbindung der Formel I in Kombination mit Omacor® (Omega-3-Fettsäuren; hochkonzentrierte Ethylester der
Eicosapentaensäure und der Docosahexaensäure) verabreicht.

Bei einer Ausführungsform der Erfindung wird die Verbindung der Formel I in Kombination mit einem ACAT-Inhibitor, wie z.B. Avasimibe oder SMP-797, verabreicht.

Bei einer Ausführungsform der Erfindung wird die Verbindung der Formel I in Kombination mit einem Antioxidans, wie z.B. OPC-14117, Probucol, Tocopherol, Ascorbinsäure, ß-Caroten oder Selen verabreicht.

Bei einer Ausführungsform der Erfindung wird die Verbindung der Formel I in Kombination mit einem Vitamin, wie z. B. Vitamin B6 oder Vitamin B12 verabreicht.

Bei einer Ausführungsform der Erfindung wird die Verbindung der Formel I in Kombination mit einem Lipoprotein-Lipase Modulator, wie z.B. Ibrolipim (NO-1886), verabreicht.

Bei einer Ausführungsform der Erfindung wird die Verbindung der Formel I in Kombination mit einem ATP-Citrat-Lyase Inhibitor, wie z.B. SB-204990, verabreicht.

Bei einer Ausführungsform der Erfindung wird die Verbindung der Formel I in Kombination mit einem Squalen Synthetase Inhibitor, wie z.B. BMS-188494 oder wie in W02005077907 beschrieben, verabreicht.

Bei einer Ausführungsform der Erfindung wird die Verbindung der Formel I in Kombination mit einem Lipoprotein(a) antagonist, wie z.B. Gemcabene (Cl-1027) verabreicht.

Bei einer Ausführungsform der Erfindung wird die Verbindung der Formel I in Kombination mit einem Aqonisten des GPR109A (HM74A Rezeptor Agonisten), wie z.B. Nicotinsäure oder "extended release niacin" in Verbindung mit MK-0524A oder solchen Verbindungen, wie sie in W02006045565, W02006045564, W02006069242 beschrieben sind, verabreicht.

Bei einer anderen Ausführungsform der Erfindung wird die Verbindung der Formel I in Kombination mit einem Aqonisten des GPR116, wie sie z.B. in W02006067531, W02006067532 beschrieben sind, verabreicht.

Bei einer Ausführungsform der Erfindung wird die Verbindung der Formel I in Kombination mit einem Lipase Inhibitor, wie z.B. Orlistat oder Cetilistat (ATL-962), verabreicht.

Bei einer Ausführungsform der Erfindung wird die Verbindung der Formel I in Kombination mit Insulin verabreicht.

Bei einer Ausführungsform wird die Verbindung der Formel I in Kombination mit einem Sulfonylharnstoff, wie z.B. Tolbutamid, Glibenclamid, Glipizid oder Glimepirid verabreicht.

Bei einer Ausführungsform wird die Verbindung der Formel I in Kombination mit einer die Insulinsekretion verstärkende Substanz, wie z. B. KCP-265 (WO2003097064), verabreicht.

Bei einer Ausführungsform wird die Verbindung der Formel I in Kombination mit Agonisten des glucose-abhängigen insulinotropischen Rezeptors (GDIR) wie z. B. APD-668 verabreicht

Bei einer Ausführungsform wird die Verbindung der Formel I in Kombination mit einem Biguanid, wie z.B. Metformin, verabreicht.

Bei wieder einer anderen Ausführungsform wird die Verbindung der Formel I in Kombination mit einem Meglitinid, wie z.B. Repaglinide oder Nateglinid, verabreicht.

Bei einer Ausführungsform wird die Verbindung der Formel I in Kombination mit einem Thiazolidindion, wie z.B. Troglitazon, Ciglitazon, Pioglitazon, Rosiglitazon oder den in WO 97/41097 von Dr. Reddy's Research Foundation offenbarten Verbindungen, insbesondere 5-[[4-[(3,4-Dihydro-3-methyl-4-oxo-2-chinazolinylmethoxy]-phenyl]methyl]-2,4-thiazolidindion, verabreicht.

Bei einer Ausführungsform wird die Verbindung der Formel I in Kombination mit einem α-Glukosidase-Inhibitor, wie z.B. Miglitol oder Acarbose, verabreicht.

Bei einer Ausführungsform wird die Verbindung der Formel I in Kombination mit einem Wirkstoff verabreicht, der auf den ATP-abhängigen Kaliumkanal der Betazellen wirkt, wie z.B. Tolbutamid, Glibenclamid, Glipizid, Glimepirid oder Repaglinid.

Bei einer Ausführungsform wird die Verbindung der Formel I in Kombination mit mehr als einer der vorstehend genannten Verbindungen, z.B. in Kombination mit einem Sulfonylharnstoff und Metformin, einem Sulfonylharnstoff und Acarbose, Repaglinid und Metformin, Insulin und einem Sulfonylharnstoff, Insulin und Metformin, Insulin und Troglitazon, Insulin und Lovastatin, etc. verabreicht.

Bei einer Ausführungsform wird die Verbindung der Formel I in Kombination mit einem Hemmstoff der Glykogenphosphorylase, wie z.B. PSN-357 oder FR-258900 oder solchen wie in W02003084922, W02004007455, W02005073229-31, W02005067932 beschrieben, verabreicht.

Bei einer Ausführungsform wird die Verbindung der Formel I in Kombination mit Glukagon-Rezeptor-Antagonisten, wie z.B. A-770077 oder NNC-25-2504 oder wie in W02004100875, W02005065680, beschrieben, verabreicht.

Bei einer Ausführungsform wird die Verbindung der Formel I in Kombination mit Aktivatoren der Glukokinase, wie z. B. LY-2121260 (WO2004063179), PSN-105, PSN-110, GKA-50 oder solchen wie sie z. B. in W02004072031, W02004072066, W02005080360, WO2005044801, W02006016194, W02006058923 beschrieben sind, verabreicht.

Bei einer Ausführungsform wird die Verbindung der Formel I in Kombination mit einem Inhibitor der Glukoneogenese, wie z. B. FR-225654, verabreicht.

Bei einer Ausführungsform wird die Verbindung der Formel I in Kombination mit Inhibitoren der Fructose-1,6-bisphosphatase (FBPase) wie z.B. CS-917 (MB-06322) oder MB-07803 oder solchen wie sie in WO2006023515 beschrieben sind, verabreicht.

Bei einer Ausführungsform wird die Verbindung der Formel I in Kombination mit Modulatoren des Glukosetransporters-4 (GLUT4), wie z. B. KST-48 (D.-O. Lee et al.: Arzneim.-Forsch. Drug Res. 54 (12), 835 (2004)), verabreicht.

Bei einer Ausführungsform wird die Verbindung der Formel I in Kombination mit Inhibitoren der Glutamin-Fructose-6-Phosphat-Amidotransferase (GFAT), wie sie z. B. in WO2004101528 beschrieben sind, verabreicht.

Bei einer Ausführungsform wird die Verbindung der Formel I in Kombination mit Inhibitoren der Dipeptidylpeptidase-IV (DPP-IV), wie z. B. Vildagliptin (LAF-237), Sitagliptin (MK-0431), Saxagliptin ((BMS-477118), GSK-823093, PSN-9301, SYR-322, SYR-619, TA-6666, TS-021, GRC-8200, GW-825964X, KRP-104, DP-893 oder wie sie in WO2003074500, WO2003106456, WO200450658, WO2005058901, WO2005012312, WO2005/012308, WO2006039325, WO2006058064, PCT/EP2005/007821, PCT/EP20051008005, PCT/EP2005/008002, PCT/EP2005/008004, PCT/EP2005/008283, DE 10 2005 012874.2 oder DE 10 2005 012873.4 beschrieben sind, verabreicht.

Bei einer Ausführungsform wird die Verbindung der Formel I in Kombination mit Januvia™, einer festen Kombination von Sitagliptin Phosphat mit Metformin hydrochlorid, verabreicht.

Bei einer Ausführungsform wird die Verbindung der Formel I in Kombination mit Hemmstoffen der 11-beta-Hydroxysteroid-Dehydrogenase-1 (11ß-HSD1), wie z. B. BVT-2733, JNJ-25918646, INCB-13739 oder solche, wie sie z. B. in WO200190090-94, WO200343999, WO2004112782, WO200344000, WO200344009, WO2004112779, WO2004113310, WO2004103980, WO2004112784, WO2003065983, WO2003104207, WO2003104208, WO2004106294, WO2004011410, WO2004033427, WO2004041264, WO2004037251, WO2004056744, WO2004058730, WO2004065351, WO2004089367, WO2004089380, WO2004089470-71, WO2004089896, WO2005016877, WO2005097759, WO2006010546, WO2006012227, WO2006012173, WO2006017542, WO2006034804, WO2006040329, WO2006051662, WO2006048750, WO2006049952, WO2006048331, WO2006050908, WO2006024627, WO2006040329, WO2006066109 beschrieben sind, verabreicht.

Bei einer Ausführungsform wird die Verbindung der Formel I in Kombination mit Inhibitoren der Protein-Tyrosin-Phosphatase-1 B (PTP1 B), wie sie z. B. in WO200119830-31, WO200117516, WO2004506446, WO2005012295, WO2005116003 PCT/EP2005/005311, PCT/EP2005/005321, PCT/EP2005/007151, PCT/EP2005101294 oder DE 10 2004 060542.4 beschrieben sind, verabreicht.

Bei einer Ausführungsform wird die Verbindung der Formel I in Kombination mit Modulatoren des natrium-abhängigen Glukosetransporters 1 oder 2 (SGLT1, SGLT2), wie z.B. KGA-2727, T-1095, SGL-0010, AVE 2268 und SAR 7226 oder wie sie z. B. in WO2004007517, WO200452903, WO200452902, PCT/EP2005/005959, WO2005085237,JP2004359630,WO2005121161,WO2006018150,WO2006035796, WO2006062224, WO2006058597 oder von A. L. Handlon in Expert Opin. Ther. Patents (2005) 15(11), 1531-1540 beschrieben sind, verabreicht.

Bei einer Ausführungsform wird die Verbindung der Formel I in Kombination mit Modulatoren des GPR40 verabreicht.

Bei einer Ausführungsform wird die Verbindung der Formel I in Kombination mit Modulatoren des GPR119b, wie sie z. B. in WO2004041274 beschrieben sind, verabreicht.

Bei einer Ausführungsform wird die Verbindung der Formel I in Kombination mit Modulatoren des GPR119, wie sie z. B. in WO2005061489 (PSN-632408) beschrieben sind, verabreicht.

Bei einer Ausführungsform wird die Verbindung der Formel I in Kombination mit Inhibitoren der hormon-sensitiven Lipase (HSL), wie z. B. in WO2005073199 beschrieben, verabreicht.

Bei einer Ausführungsform wird die Verbindung der Formel I in Kombination mit Hemmstoffen der Acetyl-CoA Carboxylase (ACC) wie z. B. solchen wie in WO199946262,WO200372197,WO2003072197, WO2005044814, WO2005108370, JP2006131559 beschrieben, verabreicht.

Bei einer Ausführungsform wird die Verbindung der Formel I in Kombination mit einem Inhibitor der Phosphoenolpyruvatcarboxykinase (PEPCK), wie z.B. solchen, wie in WO2004074288 beschrieben, verabreicht.

Bei einer Ausführungsform wird die Verbindung der Formel I in Kombination mit einem Inhibitor der Glykogen Synthase Kinase-3 beta (GSK-3 beta), wie z. B. in US2005222220, WO2005085230, PCT/EP2005/005346, WO2003078403, WO2004022544,WO2003106410, WO2005058908, US2005038023, WO2005009997, US2005026984, WO2005000836, WO2004106343, EP1460075, WO2004014910, WO2003076442, WO2005087727, WO2004046117 beschrieben.

Bei einer Ausführungsform wird die Verbindung der Formel I in Kombination mit einem Inhibitor der Protein Kinase C beta (PKC beta), wie z. B. Ruboxistaurin, verabreicht.

Bei einer Ausführungsform wird die Verbindung der Formel I in Kombination mit einem Endothelin-A-Rezeptor Antagonisten, wie z. B. Avosentan (SPP-301), verabreicht.

Bei einer Ausführungsform wird die Verbindung der Formel I in Kombination mit Inhibitoren der "l-kappaB kinase" (IKK Inhibitoren), wie sie z. B. in WO2001000610, WO2001030774, WO2004022553, WO2005097129 beschrieben sind, verabreicht.

Bei einer Ausführungsform wird die Verbindung der Formel I in Kombination mit Modulatoren des Glucocorticoidrezeptors, wie sie z. B. in WO2005090336 beschrieben sind, verabreicht.

Bei einer weiteren Ausführungsform wird die Verbindung der Formel I in Kombination mit CART-Modulatoren (siehe "Cocaine-amphetamine-regulated transcript influences energy metabolism, anxiety and gastric emptying in mice" Asakawa, A. et al.: Hormone and Metabolic Research (2001), 33(9), 554-558);
NPY-Antagonisten wie z.B. Naphthalin-1-sulfonsäure-{4-[(4-amino-quinazolin-2-ylamino)-methyl]-cyclohexylmethyl}-amid Hydrochlorid (CGP 71683A);
NPY-5 Rezeptorantagonisten wie L-152804, S-2367 oder wie sie z. B. in WO2006001318 beschrieben sind;
Peptid YY 3-36 (PYY3-36) oder analoge Verbindungen wie z. B. CJC-1682 (PYY3-36 konjugiert mit humanem Serum Albumin über Cys34) oder CJC-1643 (Derivat des
PYY3-36, welches sich in vivo an Serum Albumin konjugiert) oder solche, wie sie in WO2005080424 beschrieben sind;
CB1 R (Cannabinoid Rezeptor 1) Antagonisten (wie z.B. Rimonabant, SR147778 oder solche wie sie in z. B. EP 0656354, WO 00/15609, WO 02/076949, WO2005080345, W02005080328, WO2005080343, WO2005075450, WO2005080357, WO200170700, WO2003026647-48, WO200302776, WO2003040107, WO2003007887, WO2003027069, US6,509,367, WO200132663, WO2003086288, WO2003087037, WO2004048317, WO2004058145, WO2003084930, WO2003084943, WO2004058744, WO2004013120, WO2004029204, WO2004035566, WO2004058249, WO2004058255, WO2004058727, WO2004069838, US20040214837, US20040214855, US20040214856, WO2004096209, WO2004096763, WO2004096794, WO2005000809, WO2004099157, US20040266845, WO2004110453, WO2004108728, WO2004000817, WO2005000820, US20050009870, WO200500974, WO2004111033-34, WO200411038-39, WO2005016286, WO2005007111, WO2005007628, US20050054679, WO2005027837, WO2005028456, WO2005063761-62, WO2005061509, WO2005077897, WO2006047516, WO2006060461, WO2006067428, WO2006067443 beschrieben sind);
MC4-Agonisten (z.B. 1-Amino-1,2,3,4-tetrahydro-naphthalin-2-carbonsäure [2-(3a-benzyl-2-methyl-3-oxo-2,3,3a,4,6,7-hexahydro-pyrazolo[4,3-c]pyridin-5-yl)-1-(4-chlorophenyl)-2-oxo-ethyl]-amid; (WO 01/91752)) oder LB53280, LB53279, LB53278 oder THIQ, MB243, RY764, CHIR-785, PT-141 oder solche wie sie in WO2005060985, WO2005009950,WO2004087159,WO2004078717,WO2004078716, WO2004024720, US20050124652, WO2005051391, WO2004112793, WOUS20050222014, US20050176728, US20050164914, US20050124636, US20050130988, US20040167201, WO2004005324, WO2004037797, WO2005042516, WO2005040109, WO2005030797, US20040224901, WO200501921, WO200509184, WO2005000339, EP1460069, WO2005047253, WO2005047251, EP1538159, WO2004072076, WO2004072077, WO2006021655-57 beschrieben sind;
Orexin-Rezeptor Antagonisten (z.B. 1-(2-Methyl-benzoxazol-6-yl)-3-[1,5]naphthyridin-4-yl-harnstoff Hydrochlorid (SB-334867-A) oder solche, wie sie z. B. in WO200196302, WO200185693, WO2004085403, WO2005075458, WO2006067224 beschrieben sind);
Histamin H3 Rezeptor Agonisten (z. B. 3-Cyclohexyl-1-(4,4-dimethyl-1,4,6,7-tetrahydro-imidazo[4,5-c]pyridin-5-yl)-propan-1-on Oxalsäuresalz (WO 00/63208) oder solche, wie sie in WO200064884, WO2005082893 beschrieben sind); CRF-Antagonisten (z.B. [2-Methyl-9-(2,4,6-trimethyl-phenyl)-9H-1,3,9-triaza-fluoren-4-yl]-dipropyl-amin (WO 00/66585));
CRF BP-Antagonisten (z.B. Urocortin);
Urocortin-Agonisten;
Agonisten des beta-3 Adrenoceptors wie z.B. 1-(4-Chloro-3-methanesulfonylmethyl-phenyl)-2-[2-(2,3-dimethyl-1H-indol-6-yloxy)-ethylamino]-ethanol Hydrochlorid (WO 01/83451) oder Solabegron (GW-427353) oder N-5984 (KRP-204) oder solche, wie sie in JP2006111553 beschrieben sind;
MSH (Melanocyt-stimulierendes Hormon)-Agonisten;
MCH (melanin-konzentrierendes Hormon) Rezeptor Antagonisten (wie z. B. NBI-845, A-761, A-665798, A-798, ATC-0175, T-226296, T-71, GW-803430 oder solche Verbindungen, wie sie in WO2003/15769, WO2005085200, WO2005019240, WO2004011438, WO2004012648, WO2003015769, WO2004072025, WO2005070898, WO2005070925, WO2004039780, WO2003033476, WO2002006245, WO2002089729, WO2002002744, WO2003004027, FR2868780, WO2006010446, WO2006038680, WO2006044293, WO2006044174 beschrieben sind); CCK-A Agonisten (wie z.B. {2-[4-(4-Chloro-2,5-dimethoxy-phenyl)-5-(2-cyclohexylethyl)-thiazol-2-ylcarbamoyl]-5,7-dimethyl-indol-1-yl}-essigsäure Trifluoressigsäuresalz (WO 99/15525) oder SR-146131 (WO 0244150) oder SSR-125180) oder solchen, wie sie in WO2005116034 beschrieben sind;
Serotonin-Wiederaufnahme-Inhibitoren (z.B. Dexfenfluramine);
gemischte Sertonin- und noradrenerge Verbindungen (z.B. WO 00/71549);
5-HT-Rezeptor Agonisten z.B. 1-(3-Ethyl-benzofuran-7-yl)-piperazin Oxalsäuresalz (WO 01/09111);
5-HT2C Rezeptor Agonisten (wie z. B. Lorcaserin Hydrochlorid (APD-356) oder BVT-933 oder solche, wie sie in WO200077010, WO20077001-02, WO2005019180, WO2003064423, WO200242304, WO2005082859 beschrieben sind);
5-HT6 Rezeptor Antagonisten, wie sie z.B. in WO2005058858 beschrieben sind; Bombesin-Rezeptor Agonisten (BRS-3 Agonisten;
Galanin-Rezeptor Antagonisten;
Wachstumshormon (z.B. humanes Wachstumshormon oder AOD-9604); Wachstumshormon freisetzende Verbindungen (6-Benzyloxy-1-(2-diisopropylamino-ethylcarbamoyl)-3,4-dihydro-1 H-isochinolin-2-carbonsäuretertiärbutylester (WO 01/85695));
Growth Hormone Secretagogue Receptor Antagonisten (Ghrelin Antagonisten) wie z. B. A-778193 oder solchen, wie sie in WO2005030734 beschrieben sind;
TRH-Agonisten (siehe z.B. EP 0 462 884);
entkoppelnde Protein 2- oder 3-Modulatoren;
Leptinagonisten (siehe z.B. Lee, Daniel W.; Leinung, Matthew C.; Rozhavskaya-Arena, Marina; Grasso, Patricia. Leptin agonists as a potential approach to the treatment of obesity. Drugs of the Future (2001), 26(9), 873-881);
DA-Agonisten (Bromocriptin, Doprexin);
Lipase/Amylase-Inhibitoren (z.B. WO 00/40569);
Inhibitoren der Diacylglycerol O-Acyltransferasen (DGATs) wie z. B. BAY-74-4113 oder wie z. B. in US2004/0224997, WO2004094618, WO200058491, WO2005044250,
WO2005072740, JP2005206492, WO2005013907, WO2006004200, WO2006019020,
WO2006064189 beschrieben;
Inhibitoren der Fettsäuresynthase (FAS) wie z.B. C75 oder solchen, wie in
WO2004005277 beschrieben;
Oxyntomodulin;
Oleoyl-Estron
oder Agonisten des Schilddrüserihormonrezeptors (thyroid hormone receptor agonists) wie z. B: KB-2115 oder solche, wie in WO20058279, WO200172692, WO200194293, WO2003084915, WO2004018421, WO2005092316 beschrieben, verabreicht.

Bei einer Ausführungsform ist der weitere Wirkstoff Varenicline Tartrate, ein partieller Agonist des alpha 4-beta 2 nikotinischen Acetylcholinrezeptors.

Bei einer Ausführungsform ist der weitere Wirkstoff Trodusquemine.
Bei einer Ausführungsform ist der weitere Wirkstoff ein Modulator des Enzyms SIRT1, eines Mitglieds der humanen Sirtuinenzymfamilie.

Bei einer Ausführungsform der Erfindung ist der weitere Wirkstoff Leptin; siehe z.B. "Perspectives in the therapeutic use of leptin", Salvador, Javier; Gomez-Ambrosi, Javier; Fruhbeck, Gema, Expert Opinion on Pharmacotherapy (2001), 2(10), 1615-1622.

Bei einer Ausführungsform ist der weitere Wirkstoff Dexamphetamin oder Amphetamin.

Bei einer Ausführungsform ist der weitere Wirkstoff Fenfluramin oder Dexfenfluramin.

Bei noch einer Ausführungsform ist der weitere Wirkstoff Sibutramin.

Bei einer Ausführungsform ist der weitere Wirkstoff Mazindol oder Phentermin.

Bei einer Ausführungsform wird die Verbindung der Formel I in Kombination mit Ballaststoffen, vorzugsweise unlöslichen Ballaststoffen (siehe z.B. Carob/ Caromax^{®} (Zunft H J; et al., Carob pulp preparation for treatment of hypercholesterolemia, ADVANCES IN THERAPY (2001 Sep-Oct), 18(5), 230-6.) Caromax ist ein Carob enthaltendes Produkt der Fa. Nutrinova, Nutrition Specialties &Food Ingredients GmbH, Industriepark Höchst, 65926 Frankfurt / Main)) verabreicht. Die Kombination mit Caromax^{®} kann in einer Zubereitung erfolgen, oder durch getrennte Gabe von Verbindungen der Formel I und Caromax^{®}. Caromax^{®} kann dabei auch in Form von Lebensmitteln, wie z.B. in Backwaren oder Müsliriegeln, verabreicht werden.

Es versteht sich, dass jede geeignete Kombination der erfindungsgemäßen Verbindungen mit einer oder mehreren der vorstehend genannten Verbindungen und wahlweise einer oder mehreren weiteren pharmakologisch wirksamen Substanzen als unter den Schutzbereich der vorliegenden Erfindung fallend angesehen wird.

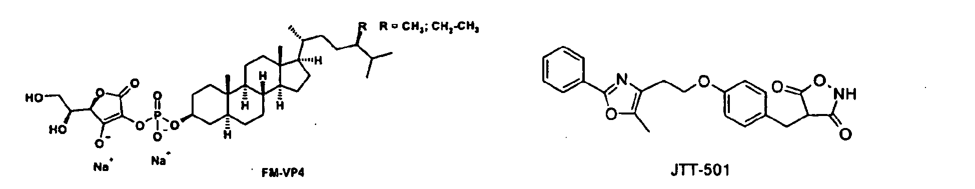

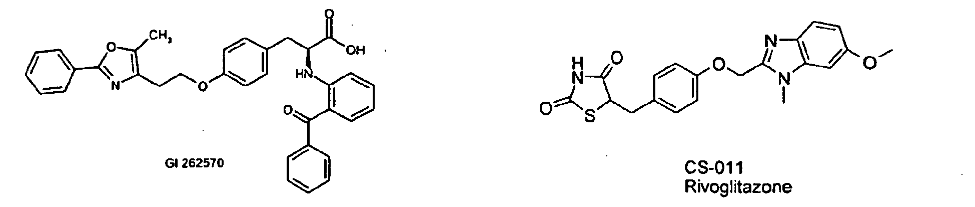

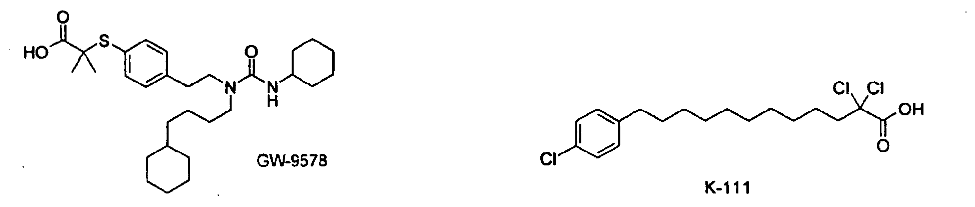

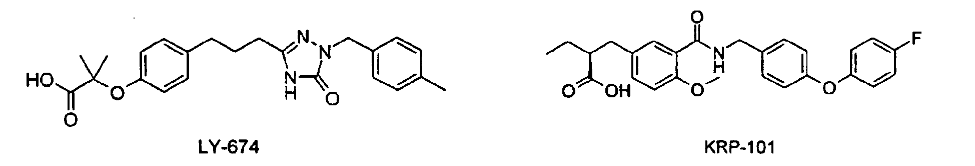

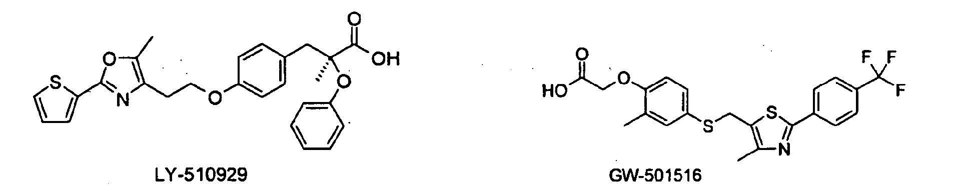

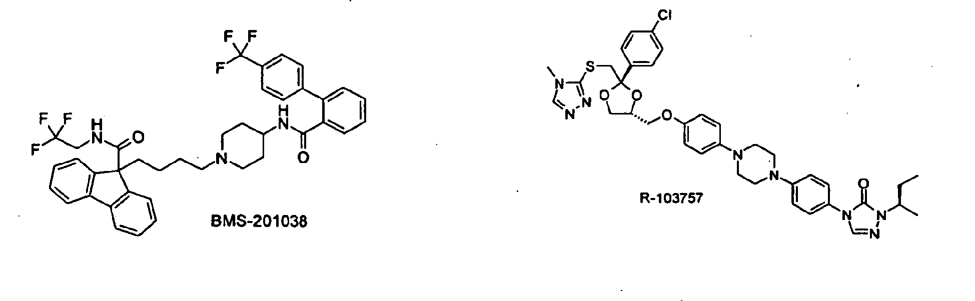

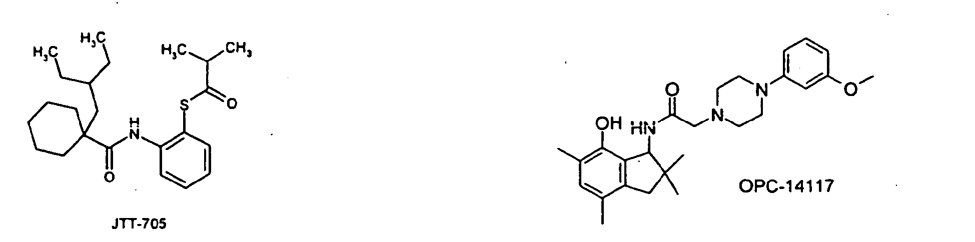

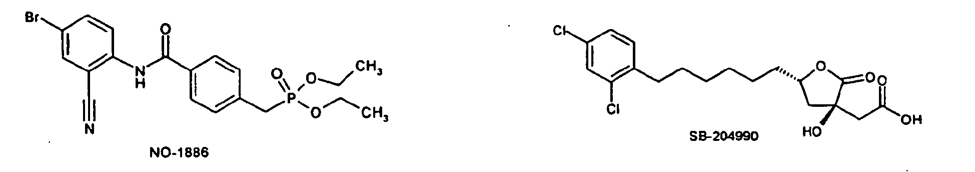

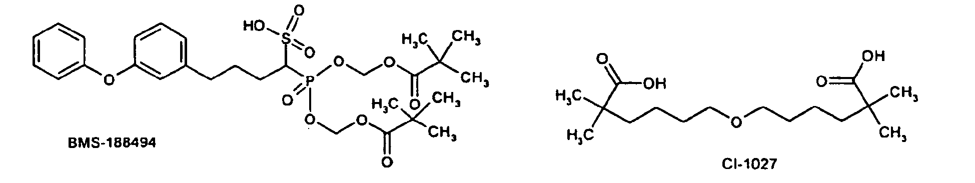

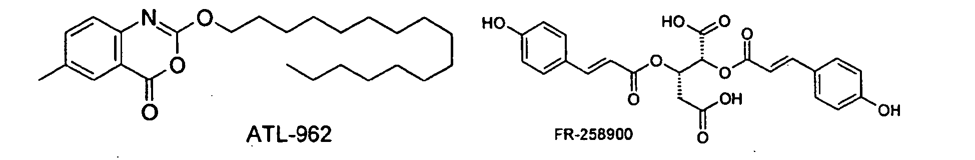

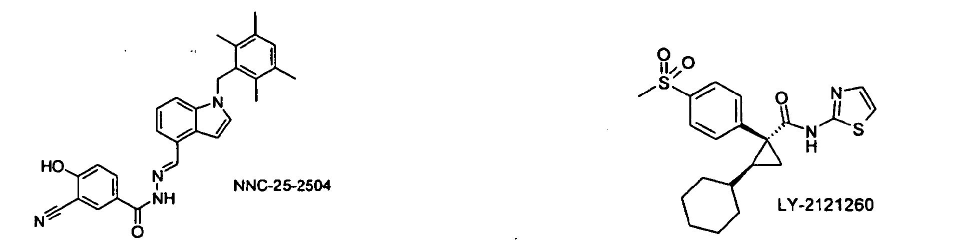

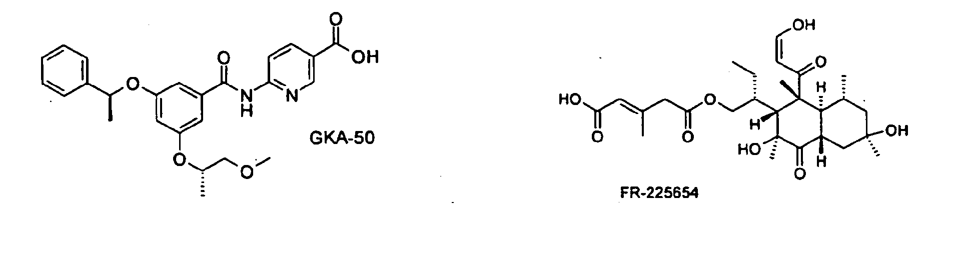

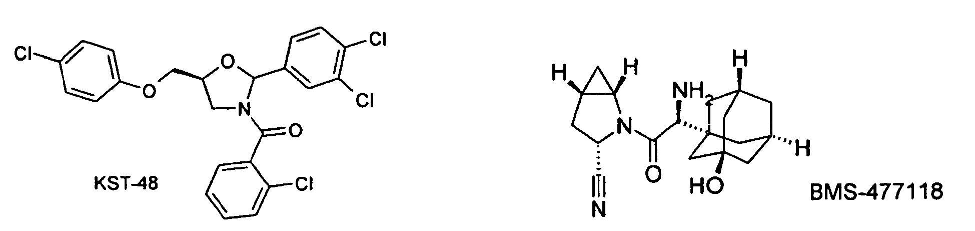

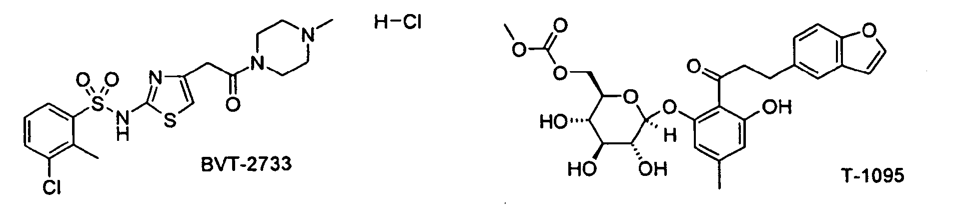

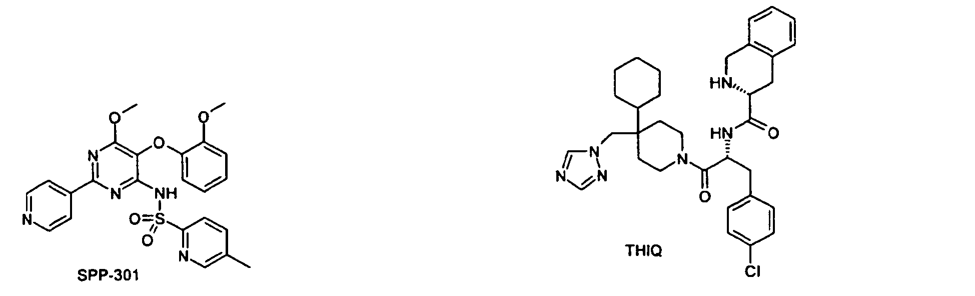

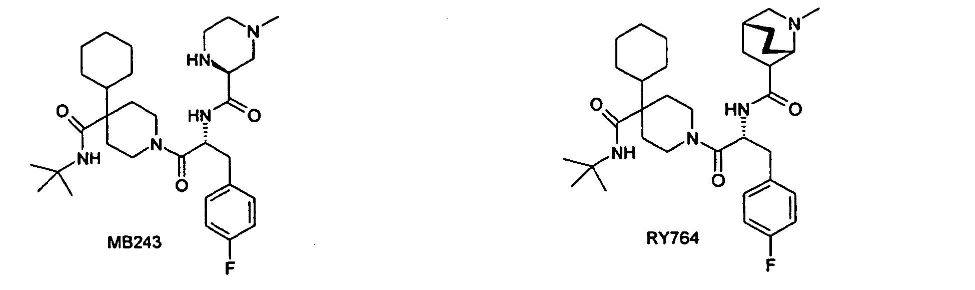

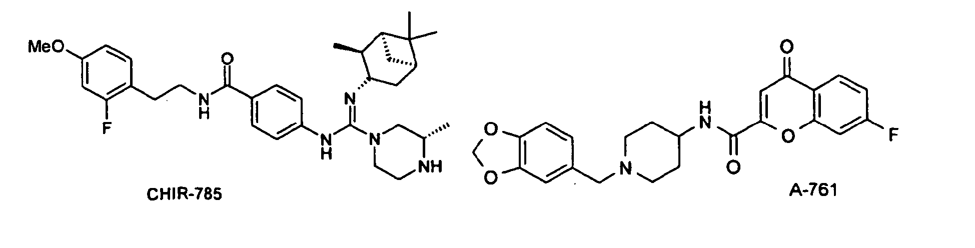

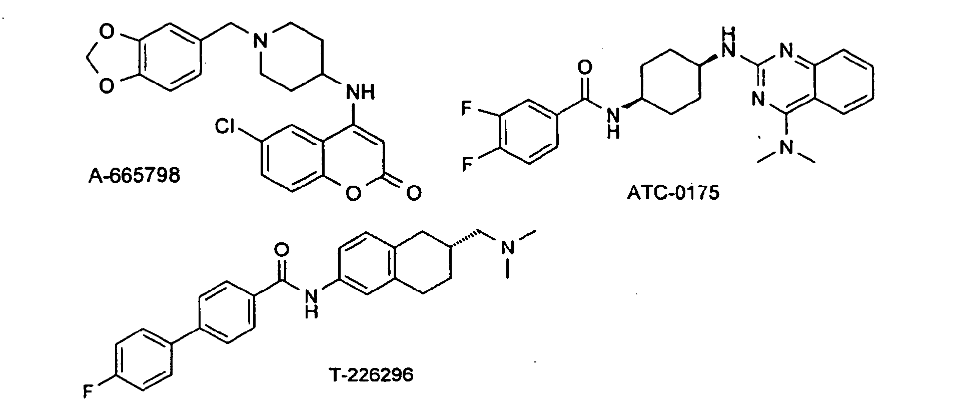

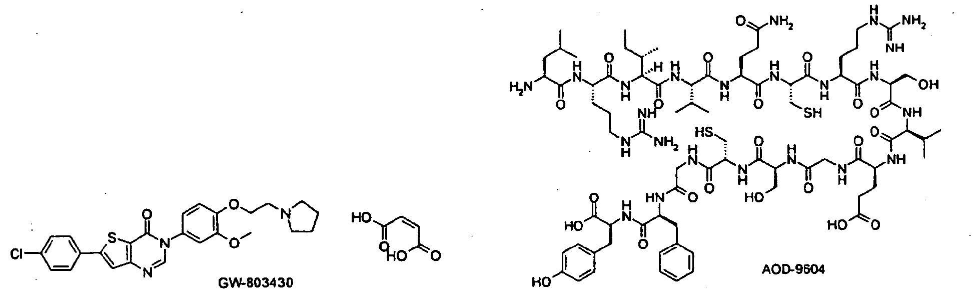

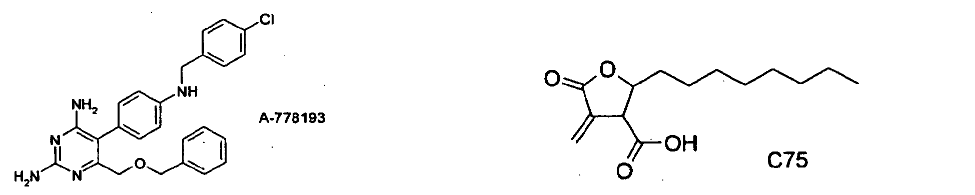

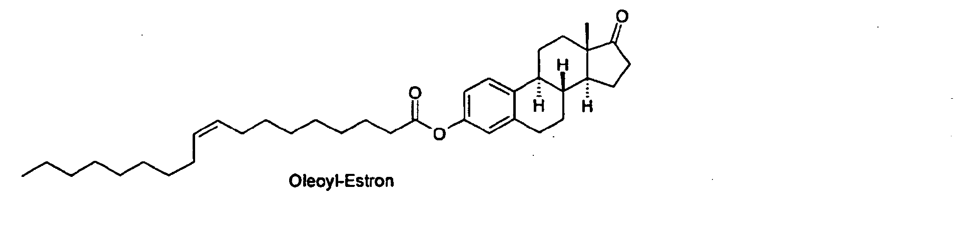

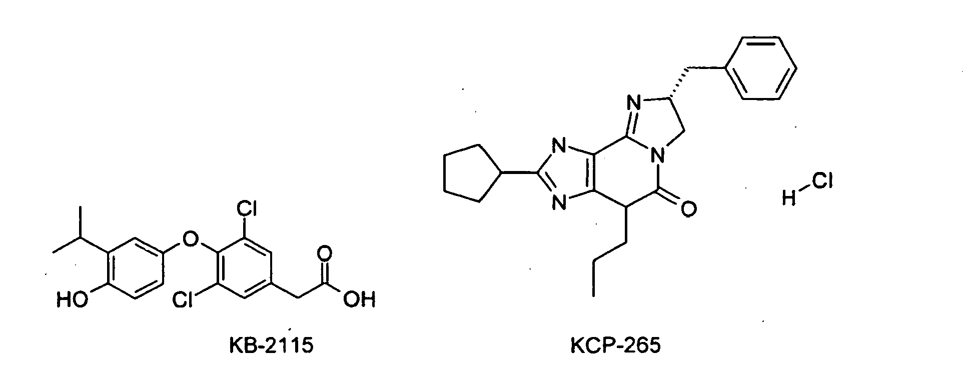

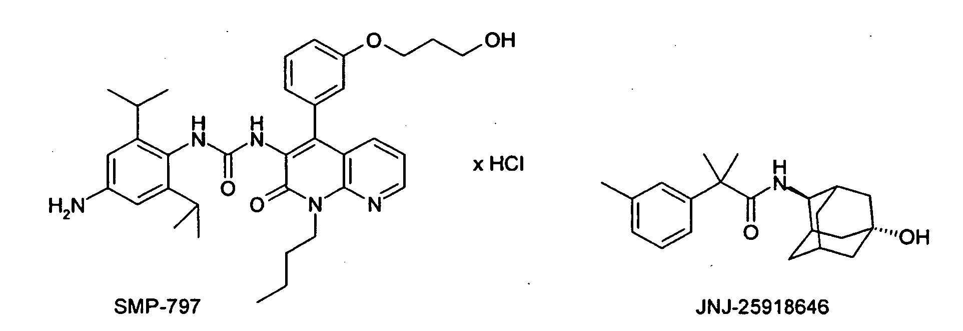

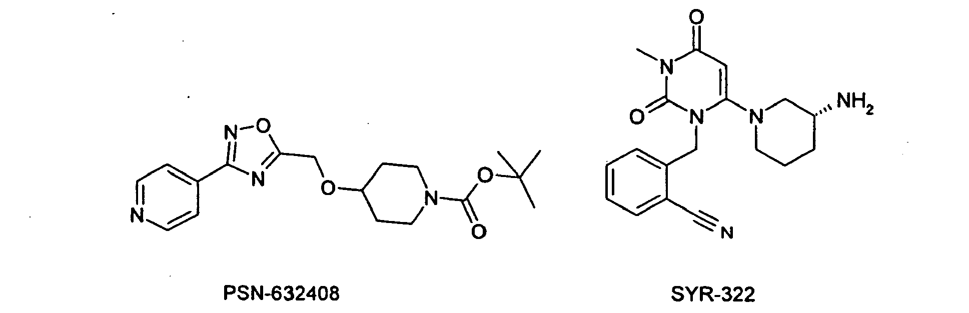

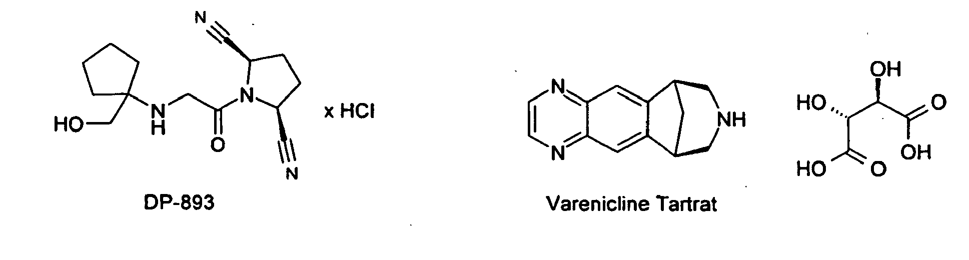

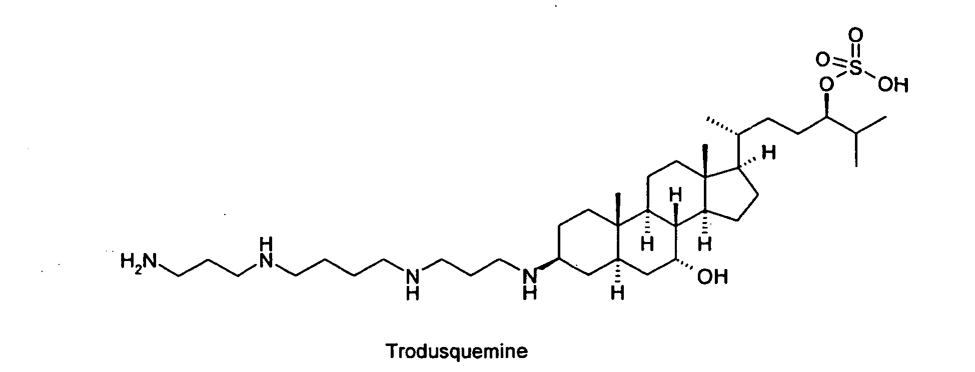

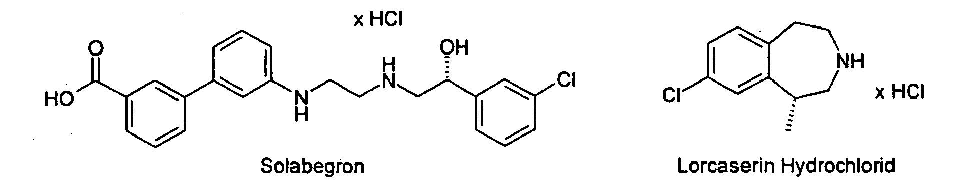

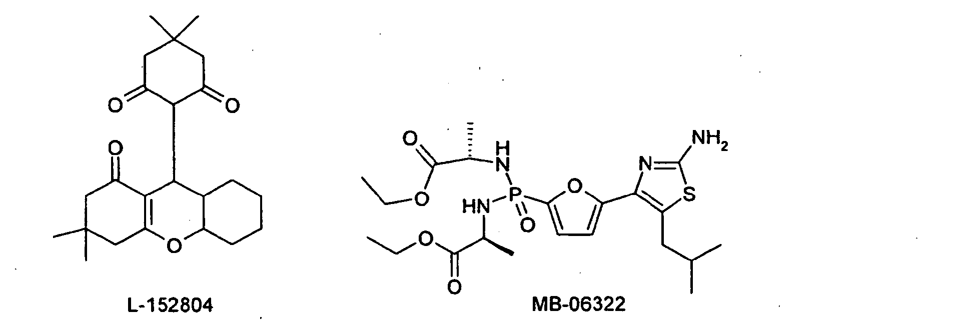

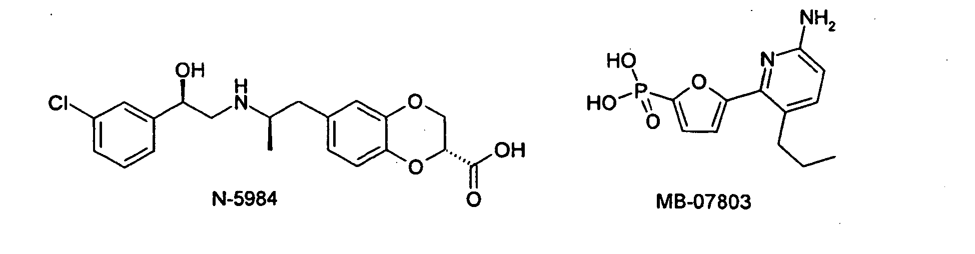

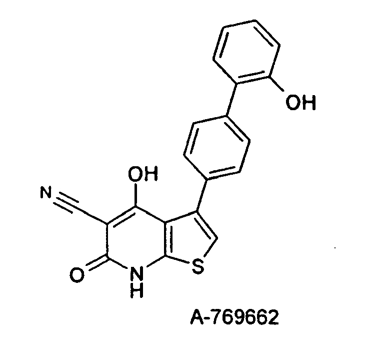

Die nachfolgend aufgeführten Beispiele dienen zur Erläuterung der Erfindung, ohne diese jedoch einzuschränken.

**Tabelle 1:**

| | | | | | | | |
|---|---|---|---|---|---|---|---|
| 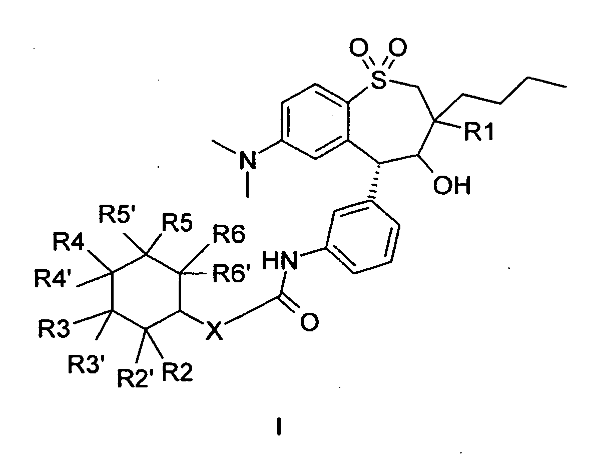 | | | | | | | |

| **Bsp** | **R1** | **R2, R2'** | **R3, R3'** | **R4, R4'** | **R5, R5'** | **R6, R6'** | **X** |
|---|---|---|---|---|---|---|---|
| 1 | Et | OH, H | OBn, H | OH, H | OBn, H | OH, H | NH |
| 2 | Et | OSO₂OH, H | OBn, H | OSO₂OH, H | OBn, H | OSO₂OH, H | NH |
| 3 | Et | OH, H | OH, H | OH, H | OH, H | OH, H | NH |

| | | | | | | | |
|---|---|---|---|---|---|---|---|
| Bn = Benzyl, Et = Ethyl | | | | | | | |

Die Wirksamkeit der Verbindungen wurde wie folgt getestet:
Vorarbeiten und Durchführung des *in vitro* IBAT Hemmtestes:
   1. Klonierung eines Expressionsvektors für den humanen IBAT
      Die cDNA (complementary deoxyribonucleic acid) des humanen IBAT wurde über molekularbiologische Standardmethoden, wie z.B. in Molecular Cloning: A Laboratory Manual von Joseph Sambrook und David Russell beschrieben, kloniert und in den pcDNA1 Vektor der Fa. Invitrogen eingebracht. Die anschließende Sequenzierung des Inserts ergab vollständige Identität mit den Basen 599 bis 1645 der von P.A. Dawson beschriebenen und in der GenBank Sequenzdatenbank hinterlegten Basensequenz für den humanen IBAT (GenBank Accesion Nummer: U10417). Die Basen 599 bis 1645 entsprechen der kompletten kodierenden Region des humanen IBAT.
   2. Herstellung einer rekombinanten Zelllinie mit konstitutiver Expression des humanen IBAT
      Der Expressionsvektor für den humanen IBAT wurde mittels stabiler Transfektion in CHO (chinese hamster ovary) Zellen eingeführt. Zur Selektion von Einzelzellklonen wurde dem Zellkulturmedium (Ham's F12 Medium supplementiert mit 10% fötalem Kälberserum, 100 Einheiten/ml Penicillin, 100 Einheiten/ml Streptomycin) 400µp/ml Geneticin zugesetzt. Die Funktionalität der aus der Selektion resultierenden Einzelzellklone wurde über deren Aufnahmeaktivität für radioaktiv markierte Taurocholsäure ([³H]-TCA) getestet. Derjenige Zellklon mit der höchsten Aufnahmeaktivität für [³H]-TCA, nachfolgend CHO-hlBAT bezeichnet, wurde für die weiteren Tests ausgewählt und weiterhin in Anwesenheit von 400µg/ml Geneticin kultiviert.
   3. Messung der hemmenden Wirkung der erfindungsmäßigen Verbindung auf die IBAT-abhängige Aufnahme von Taurocholsäure in Zellen CHO-hlBAT Zellen wurden in einer Konzentration von 40 000 Zellen pro Well in Poly-D-Lysin-beschichtete 96-Well Platten in Zellkulturmedium ausgesät und für 24 h kultiviert. Dann wurden die Zellen einmal mit natrium-freiem Transport-Assay-Puffer (140mM Cholinchlorid, 2mM Kaliumchlorid, 1mM Magnesiumchlorid, 1mM Kalziumchlorid, 10mM HEPES/Tris, pH7,5) gewaschen und anschließend entweder mit natrium-freiem Transport-Assay-Puffer als Negativkontrolle oder mit natrium-haltigem Transport Assay Puffer (140mM Natriumchlorid, 2mM Kaliumchlorid, 1 mM Magnesiumchlorid, 1 mM Kalziumchlorid, 10mM HEPES/Tris, pH7,5) als Positivkontrolle für 30 min bei Raumtemperatur inkubiert. Gleichzeitig wurden auch die Testwells in Anwesenheit der zu untersuchenden Verbindung in unterschiedlicher Konzentration für 30 min bei Raumtemperatur in natrium-haltigem Transport-Assay-Puffer inkubiert. Die Testsubstanzen wurden ausgehend von einer 10mM Stammlösung in Dimethylsulfoxid entsprechend in Transport-Assay-Puffer verdünnt (40µl/Well). Der Test wurde anschließend durch Zugabe von 10µl/Well einer Mischung aus radioaktiv markierter Taurocholsäure ([³H]-TCA) und unmarkierter Taurocholsäure gestartet. Die Endkonzentration der Taurocholsäure im Test lag bei 10µM. Nach einer Inkubationszeit von 60 min bei Raumtemperatur wurde die Reaktion durch Zugabe von 100µl/Well natrium-freiem Transport-Assay-Puffer (4°C) gestoppt und jedes Well dreimal mit natrium-freiem Transport-Assay-Puffer gewaschen. Zuletzt wurde jedem Well 100µl Szintillationsflüssigkeit zugegeben und die in die Zellen aufgenommene Radioaktivität in einem MicroBeta Scintillation Microplate Reader der Fa. Wallac bestimmt.

Die halbmaximale Hemmwirkung der Testverbindung (IC50 Wert, inhibitory concentration 50) wurde folgendermaßen bestimmt:
1. Feststellung des Wertes für 0% Inhibition. Dies ist der Messwert bei Abwesenheit von Substanz, gemessen in natrium-haltigem Transport-Assay-Puffer.
2. Feststellung des Wertes für 100% Inhibition. Dies ist der Messwert bei Abwesenheit von Substanz, gemessen in natrium-freiem Transport-Assay-Puffer.
3. Errechnung der prozentualen Hemmwerte derjenigen Messungen, die in Anwesenheit unterschiedlicher Konzentrationen der zu untersuchenden Verbindung durchgeführt wurden. Daraus konnte dann diejenige Konzentration der Verbindung ermittelt werden, welche die Aufnahme der Taurocholsäure um 50% reduziert (IC50 Wert).

**Tabelle 2: Biologische Aktivität**

| Bsp. | IC-50 (human IBAT) µM |
|---|---|
| 1 | 0,0042 |
| 2 | 0.016 |

Aus den Messdaten ist abzulesen, dass die erfindungsgemäßen Verbindungen der Formel I gut zur Behandlung Behandlung von Hyperlipidämie geeignet sind.

Die Verwendung der Verbindungen der Formel I zur Behandlung bzw. Prävention weiterer Krankheiten, ist ebenfalls denkbar. Solche Krankheiten sind zum Beispiel:
1.
   - Störungen des Fettsäurestoffwechsels und Glucoseverwertungsstörungen
   - Störungen, bei denen Insulinresistenz eine Rolle spielt
2. Diabetes mellitus, insbesondere Typ-2-Diabetes, einschließlich der Prävention der damit verbundenen Folgeerkrankungen.
   - Besondere Aspekte in diesem Zusammenhang sind
   - Hyperglykämia,
   - Verbesserung der Insulinresistenz,
   - Verbesserung der Glucosetoleranz,
   - Schutz der ß-Zellen der Bauchspeicheldrüse
   - Prävention makro- und mikrovaskulärer Erkrankungen
3. Dyslipidämien und deren Folgen wie beispielsweise Atherosklerose, koronare Herzkrankheit, zerebrovaskuläre Erkrankungen usw., insbesondere (jedoch nicht darauf beschränkt) die, die durch einen oder mehrere der folgenden Faktoren gekennzeichnet sind:
   - hohe Plasmatriglyceridkonzentrationen, hohe postprandiale Plasmatriglyceridkonzentrationen,
   - niedrige HDL-Cholesterinkonzentration
   - niedrige ApoA-Lipoproteinkonzentrationen
   - hohe LDL-Cholesterinkonzentrationen
   - kleine dichte LDL-Cholesterinpartikel
   - hohe ApoB-Lipoproteinkonzentrationen
   - Desaturierungsindex (z.B. Verhältnis 18:1/18:0n-9, 16:1/16:0 n-7 oder 18:1 n-9 + 16:1 n-7 /16:0 Fettsäuren)
4. Verschiedene andere leiden, die mit dem metabolischen Syndrom bzw. Syndrom X assoziert sein können, wie
   - Zunahme des Bauchumfangs
   - Dyslipidämie (z.B. Hypertriglyceridämie und/oder niedriges HDL)
   - Insulinresistenz
   - Hyperkoagulabilität
   - Hyperurikämie
   - Mikroalbuminämie
   - Thrombosen, hyperkoagulabile und prothrombotische Zustände (arteriell und venös)
   - Bluthochdruck
   - Herzinsuffizienz, beispielsweise (jedoch nicht darauf beschränkt), nach Herzinfarkt, hypertensiver Herzkrankheit oder Kardiomyopathie
5. Leberstörungen und damit in Zusammenhang stehende Leiden
   - Fettleber
   - hepatische Steatose
   - nicht alkoholbedingte Hepatitis
   - nicht alkoholbedingte Steatohepatitis (NASH)
   - alkoholbedingte Hepatitis
   - akute Fettleber
   - schwangerschaftsbedingte Fettleber
   - arzneimittelinduzierte Hepatitis
   - Eisenspeicherkrankheiten
   - hepatische Fibrose
   - hepatische Zirrhose
   - Hepatom
   - virale Hepatitis
6. Hauterkrankungen und -leiden bzw. solche, die mit mehrfach ungesättigten Fettsäuren in Zusammenhang stehen
   - Ekzem
   - Akne
   - Schuppenflechte
   - Bildung bzw. Prävention von Wutstnarben
   - andere mit der Fettsäurezusammensetzung der Schleimhautmembran in Zusammenhang stehende Krankheiten
7. Primäre Hypertriglyceridämie oder sekundäre Hypertriglyceridämien nach
   - familiärer Retikulohistiozytose
   - Lipoproteinlipasemangel
   - Hyperlipoproteinämien
   - Apolipoproteinmangel (z.B. Apocll- oder ApoE-Mangel)
8. Krankheiten bzw. Leiden, die mit neoplastischer zellulärer Proliferation in Zusammenhang stehen
   - gutartige oder maligne Tumore
   - Krebs
   - Neoplasien
   - Metastasen
   - Karzinogenese
9. Krankheiten bzw. Leiden, die mit neurologischen, psychiatrischen oder Immunerkrankungen bzw. -leiden in Zusammenhang stehen
10. Andere Krankheiten bzw. Leiden, an denen beispielsweise entzündliche Reaktionen oder Zelldifferenzierung beteiligt sein können, sind:
   - Atherosklerose wie beispielsweise (jedoch nicht darauf beschränkt) Koronarsklerose einschließlich Angina pectoris oder Herzinfarkt, Schlaganfall, ischämischer Schlaganfall und transitorische ischämische Attacke (transient ischemic attack, TIA)
   - periphere Verschlußkrankheit
   - vaskuläre Restenose oder Reokklusion
   - chronische entzündliche Darmerkrankungen wie beispielsweise Morbus Crohn und Colitis ulcerosa
   - Pankreatitis
   - Sinusitis
   - andere entzündliche Leiden
   - Retinopathie, ischämische Retinopathie
   - Fettzellentumore
   - lipomatöse Karzinome wie beispielsweise Liposarkome
   - feste Tumore und Neoplasmen wie beispielsweise (jedoch nicht darauf beschränkt) Karzinome des Magen-Darm-Trakts, der Leber, der Gallenwege und der Bauchspeicheldrüse, endokrine Tumore, Karzinome der Lunge, der Niere und der ableitenden Harnwege, des Genitaltrakts, Prostatakarzinome usw.
   - akute und chronische myeloproliferative Erkrankungen und Lymphome
   - Angiogenese
   - neurodegenerative Erkrankungen
   - Alzheimer-Krankheit
   - multiple Sklerose
   - Parkinson-Krankheit
   - erythematosquamöse Dermatosen wie beispielsweise Schuppenflechte
   - Acne vulgaris
   - andere Hauterkrankungen und dermatologische Leiden, die durch PPAR moduliert werden
   - Ekzeme und Neurodermatitis
   - Dermatitis wie beispielsweise seborrhoische Dermatitis oder Lichtdermatitis
   - Keratitis und Keratosen wie beispielsweise seborrhoische Keratosen, senile Keratosen, aktinische Keratoses, lichtinduzierte Keratosen oder Keratosis follicularis
   - Wulstnarben und Wulstnarbenprophylaxe
   - Warzen einschließlich Kondylomen oder Condylomata acuminata
   - Infektionen mit dem humanen Papillomavirus (HPV) wie beispielsweise venerische Papillome, von Viren verursachte Warzen wie beispielsweise Molluscum contagiosum, Leukoplakie
   - papulöse Dermatosen wie beispielsweise Lichen planus
   - Hautkrebs wie beispielsweise Basalzellenkarzinome, Melanome oder kutane T-Zellenlymphome
   - lokalisierte gutartige epidermale Tumore wie beispielsweise Keratoderma, epidermale Naevi
   - Frostbeulen
   - Bluthochdruck
   - Syndrom X
   - Symdrom der polyzystischen Ovarien (polycystic ovary syndrome, PCOS)
   - Asthma
   - zystische Fibrose
   - Osteoarthritis
   - Lupus erythematodes (LE) oder entzündliche rheumatische Erkrankungen wie beispielsweise rheumatoide Arthritis
   - Gefäßentzündung
   - Auszehrung (Kachexie)
   - Gicht
   - Ischämia/Reperfusionssyndrom
   - Schocklunge (acute respiratory distress syndrome, ARDS)
   - Virenerkrankungen und -infektionen
   - Lipodystrophie und lipodystrophische Leiden, auch bei Arzneimittelnebenwirkungen (z.B. nach der Einnahme von Medikamenten zur Behandlung von HIV oder Tumoren)
   - Myopathien und Lipidmyopathien (wie Carnitinpalmitoyltransferase-I- oder-II-Mangel)

Nachfolgend wird die Herstellung einiger Beispiele detailliert beschrieben, die übrigen Verbindungen der Formel I wurden analog erhalten:
Experimenteller Teil:
   Beispiel **1** 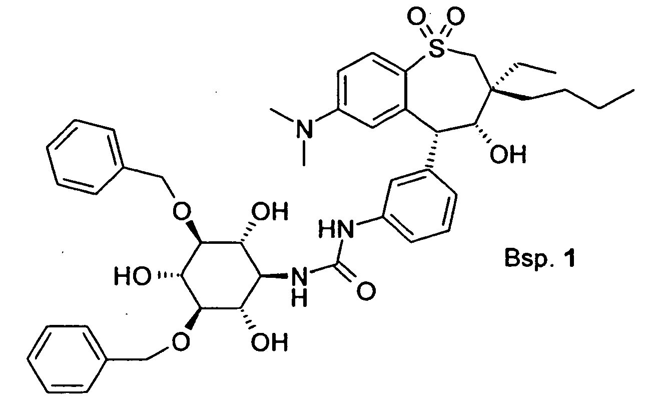 Synthese des Inositolisocyant **5:** 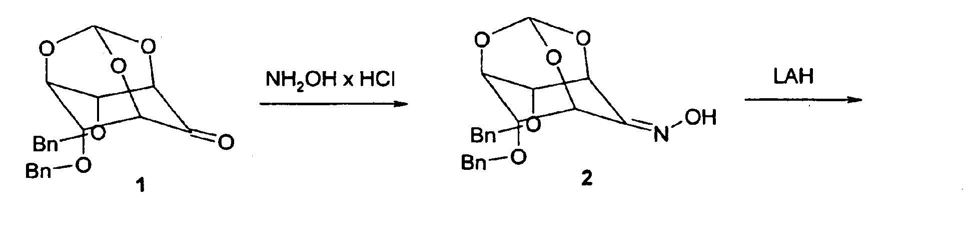 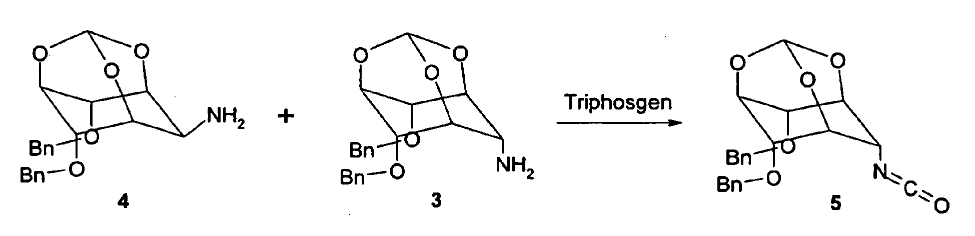 8.4 g (22.8 mmol) Inositolketon **1** (J. of Organic Chemistry (1985), 50(22), 4402-4404) werden in 180 ml Pyridin gelöst. Nach Zugabe von 18.5 g Hydroxylamin-hydrochlorid wird 4 Stunde am Rückfluß gekocht. Die Reaktionslösung wird auf Raumtemperatur gekühlt und mit Wasser und Ethylacetat extrahiert, die organische Phase noch zweimal mit gesättigter Natriumchloridlösung gewaschen, über wenig Kieselgel filtriert und eingeengt. Der Rückstand wird mit Flashchromatographie gereinigt. Man erhält 5.45 g farbloser Festoff **2** (62 % Ausbeute). DC (n-Heptan/Ethylacetat 2/1). R_{f} = 0.5. C₂₁H₂₁NO₆ (383.40). MS (M+H)⁺ = 384.03.
      Synthese von Verbindung **3** und **4:**
      5.2 g (13.6 mmol) Imin **2** werden in 50 ml Tetrahydrofuran gelöst. Dann wird vorsichtig 50 ml 1 M Litiumaluminiumhydrid/THF-Lösung (LAH) zugetropft und anschließend 2 Stunden am Rückfluß gekocht. Die Reaktionslösung wir auf 0 °C gekühlt und mit Ethylacetat zerstört man vorsichtig überschüssigen LAH. Um einen gut filtrierbaren Niederschlag zu bekommen gibt man nacheinander vorsichtig 2.2 ml Wasser, 2.2 ml 10 % Natronlauge und dann nochmals 2.2 ml Wasser zu, Der Niederschlag wird dann über wenig Kieselgel abgesaugt und das Filtrat eingeengt. Der Rückstand wird mit Flashchromatographie gereinigt. Man erhält 2.91 g farbloser Festoff **4** (58 % Ausbeute) und 0.72 g (14 %) Amin **3.** DC (n-Heptan/Ethylacetat 1/1). R_{f}= 0.1 für Amin **3** und R_{f}= 0.4 für Amin **4.** C₂₁H₂₃NO₅ (369.42). MS (M+H)⁺ = 370.18. Die all-äquartorialle Struktur vom Inositolteil der Beispiele **1** bis **3** wurde mittels NMR Spektroskopie bestättigt (scyllo-Inositol Amin **4** und myo-Inositol Amin **3).**
      Synthese von Verbindung **5**:
      1.5 g (4.1 mmol) Amin-derivat **4** werden in 50 ml Methylenchlorid gelöst. Nach Zugabe von 3.6 ml Triethylamin wird auf 0 °C gekühlt und 1.8 g (6.7 mmol) Triphosgen (Aldrich) zugegeben. Man läst 60 Minuten bei 0 °C reagieren. Die Lösung wird anschliessend mit Ethylacetat verdünnt und über wenig Kieselgel filtriert, eingeengt und man erhält 1.9 g Rohprodukt Isocyanat **5** als farbolses Öl, das ohne weitere Reinigung weiter umgesetzt wird (Isocyanat **5** zersetz sich beim chromatographieren). 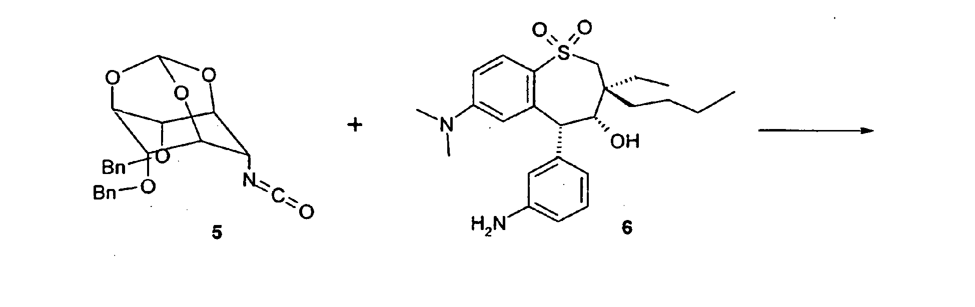 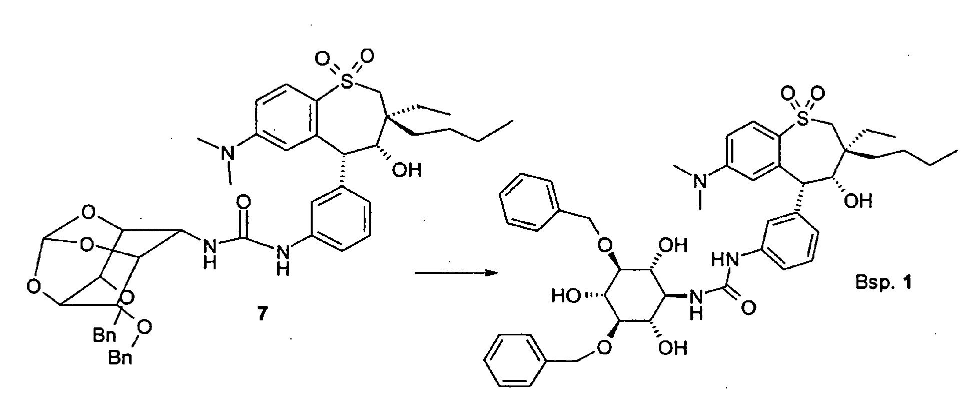 Synthese von Verbindung **7**:
      3.5 g (8.1 mmol) Anilid **6** (US 5.994.391) und 1.9 g (maximal 4.1 mmol) Isocyanat **5** (Rohprodukt) werden in 60 ml Methylenchlorid bei Raumtemperatur gelöst. Nach 3
      Stunden bei 40°C wird eingeengt und man erhält 5.4 g Rohprodukt **7** als farbloser Feststoff. DC (n- Heptan/ Ethylacetat 1:2). R_{f} = 0.3.
      Synthese von Beispiel **1:**
      5.4 g Rohprodukt **7** werden in 50 ml 0.5 M HCl/Methanol gelöst und 1 Stunde bei 50 °C gerührt. Das Methanol wird am Rotationsverdampfer eingeengt, der Rückstand wird noch einmal mit 10 ml Methanol abgeraucht und dann mit Flashchromatographie gereinigt. Ausbeute 1.22 g (36 % über 2 Stufen) Bsp. **1.** DC (Methylenchlorid/
      Methanol/ konz. Ammoniak 100/7/1). R_{f}= 0.8. C₄₅H₅₇N₃O₉S (816.03). MS (M + H)⁺= 816.53.
   Beispiel **2** 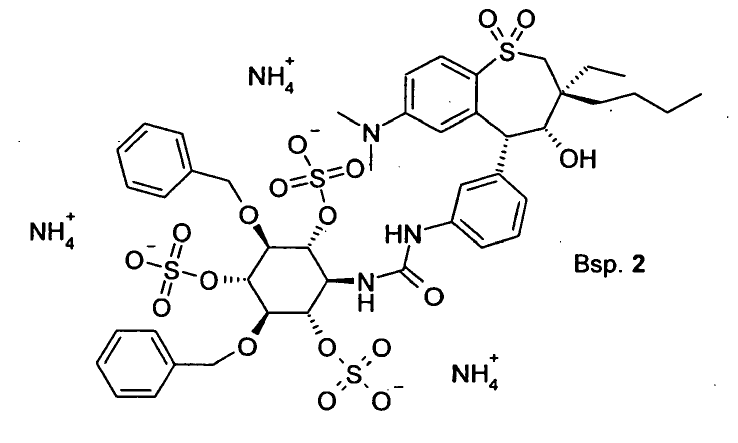 50.0 mg (0.061 mmol) Beispiel **1** wird in 3 ml Pyridin gelöst und nach Zugabe von 150 mg Trimethylamin-schwefeltrioxid-komplex 20 Stunden bei Raumtemperatur gerührt. Nach Zugabe von 10 ml Methanol wird am Rotationsverdampfer eingeengt. Der Rückstand wird noch einmal mit 10 ml Methanol abgeraucht und dann mit Flashchromatographie gereinigt. Ausbeute 42 mg (62 %) Bsp. **2** als Tri-Ammoniumsalz. DC (Methylenchlorid/ Methanol/ konz. Ammoniak 30/15/5). R_{f}= 0.4. C₄₅H₅₇N₃O₁₈S₄ x 3NH₃ (1107.31). MS (M + H)⁺ = 1056.24.
   Beispiel **3** 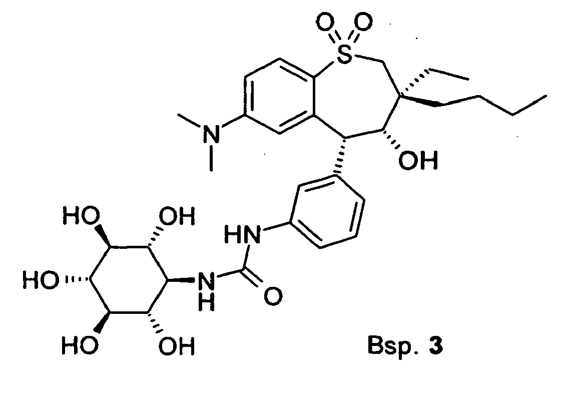 105 mg (0.13 mmol) Beispiel **1** werden in 3 ml Methylenchlorid und 3 ml Methanol gelöst und mit 0.5 ml 0.5 M HCl/Methanol versetzt. Nach Zugabe von 30 mg Palladium auf Aktivkohle (10 % Pd) wird 5 Stunden bei 5 bar Wasserstoffdruck hydriert. Die Palladium-kohle wird mit Hilfe eines Spritzenfilters abgetrennt und man erhält nach entfernen des Lösungsmittel 80 mg (97 %) Beispiel **3** als farbloser Feststoff. DC (Methylenchlorid/ Methanol/ konz. Ammoniak 30/10/3). R_{f}= 0.3. C₃₁H₄₅N₃O₉S (635.78). MS (M + H)⁺ = 636.30.

## Patentansprüche

1. Verbindungen der Formel I, 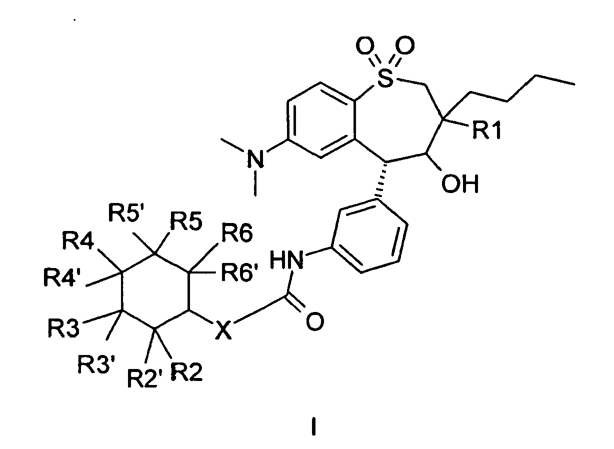 worin bedeuten
X CH₂, O, NH;
R1 (C₁-C₄)-Alkyl;
R2, R2', R3, R3', R4, R4', R5, R5', R6, R6' unabhängig voneinander H, OH, F, CI, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; C(=NH)(NH₂), PO₃H₂, SF₅, SO₃H, OSO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, CL, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
sowie deren pharmazeutisch verträgliche Salze.

2. Verbindungen der Formel I, gemäß Anspruch 1, **dadurch gekennzeichnet, dass** darin bedeuten
X O, NH;
R1 (C₁-C₄)-Alkyl;
R2, R2', R3, R3', R4, R4', R5, R5', R6, R6' unabhängig voneinander H, OH, F, Cl, Br,l, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; C(=NH)(NH₂), PO₃H₂, SF₅, SO₃H, OSO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann;
NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br,I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
sowie deren pharmazeutisch verträgliche Salze.

3. Verbindungen der Formel I, gemäß Anspruch 1 oder 2, **dadurch gekennzeichnet, dass** darin bedeuten
X NH;
R1 (C₁-C₄)-Alkyl;
R2, R2', R3, R3', R4, R4', R5, R5', R6, R6' unabhängig voneinander H, OH, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; C(=NH)(NH₂), PO₃H₂, SF₅, SO₃H, OSO₃H, SO₂-NH₂, SO₂NH(C₁-C₆)-Alkyl, SO₂N[(C₁-C₆)-Alkyl]₂ , S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br,l, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
sowie deren pharmazeutisch verträgliche Salze.

4. Verbindungen der Formel I, gemäß gemäß einem oder mehreren der Ansprüche 1 bis 3, **dadurch gekennzeichnet, dass** darin bedeuten
X NH;
R1 (C₁-C₄)-Alkyl;
R2', R3', R4', R5', R6' H;
R2, R3, R4, R5, R6 unabhängig voneinander OH, F, Cl, Br, I, CF₃, NO₂, N₃, CN, COOH, COO(C₁-C₆)Alkyl, CONH₂, CONH(C₁-C₆)Alkyl, CON[(C₁-C₆)Alkyl]₂, (C₁-C₆)-Alkyl, (C₂-C₆)-Alkenyl, (C₂-C₆)-Alkinyl, O-(C₁-C₆)-Alkyl, wobei in den Alkylresten ein, mehrere, oder alle Wasserstoff(e) durch Fluor ersetzt sein können; C(=NH)(NH₂), PO₃H₂, SF₅, SO₃H, OSO₃H, SO₂-NH₂, SO₂NH(C₁-C₆)-Alkyl, SO₂N[(C₁-C₆)-Alkyl]₂, S-(C₁-C₆)-Alkyl, S-(CH₂)ₙ-Phenyl, SO-(C₁-C₆)-Alkyl, SO-(CH₂)ₙ-Phenyl, SO₂-(C₁-C₆)-Alkyl, SO₂-(CH₂)ₙ-Phenyl, wobei n = 0 - 6 sein kann und der Phenylrest bis zu zweifach mit F, Cl, Br, OH, CF₃, NO₂, CN, OCF₃, O-(C₁-C₆)-Alkyl, (C₁-C₆)-Alkyl, NH₂ substituiert sein kann; NH₂, NH-(C₁-C₆)-Alkyl, N((C₁-C₆)-Alkyl)₂, NH(C₁-C₇)-Acyl, Phenyl, O-(CH₂)ₙ-Phenyl, wobei n = 0 - 6 sein kann, wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)2, SO₂-CH₃, COOH, COO-(C₁-C₆)-Alkyl, CONH₂;
sowie deren pharmazeutisch verträgliche Salze.

5. Verbindungen der Formel I, gemäß gemäß einem oder mehreren der Ansprüche 1 bis 3, **dadurch gekennzeichnet, dass** darin bedeuten
X NH;
R1 (C₁-C₄)-Alkyl;
R2', R3', R4',. R5', R6' H;
R2, R3, R4, R5, R6 unabhängig voneinander OH, O-(C₁-C₆)-Alkyl, wobei in den Alkylresten ein, mehrere, oder alle Wasserstoff(e) durch Fluor ersetzt sein können; OSO₃H, NH₂, NH-(C₁-C₆)-Alkyl, N((C₁-C₆)-Alkyl)2, O-(CH₂)ₙ-Phenyl, wobei n = 0 - 6 sein kann und wobei der Phenylring ein bis 3-fach substituiert sein kann mit F, Cl, Br, I, OH, CF₃, NO₂, CN, OCF₃, O-(C₁-C₆)-Alkyl, (C₁-C₆)-Alkyl, NH₂, NH(C₁-C₆)-Alkyl, N((C₁-C₆)-Alkyl)₂, SO₂-CH₃, COOH, COO-(C₁-C₆)-Alkyl, CONH₂;
sowie deren pharmazeutisch verträgliche Salze.

6. Verbindungen der Formel I, gemäß einem oder mehreren der Ansprüche 1 bis 5, zur Anwendung als Arzneimittel.

7. Arzneimittel enthaltend Verbindungen der Formel I, gemäß einem oder mehreren der Ansprüche 1 bis 5.

8. Arzneimittel enthaltend Verbindungen der Formel I, gemäß einem oder mehreren der Ansprüche 1 bis 5 und mindestens einen weiteren Wirkstoff.

9. Arzneimittel, gemäß Anspruch 8, **dadurch gekennzeichnet, daß** es als weiteren Wirkstoff eine oder mehrere Verbindungen, die den Lipidstoffwechsel normalisieren, enthält.

10. Arzneimittel, gemäß Anspruch 9, **dadurch gekennzeichnet, dass** es als weiteren Wirkstoff eine oder mehrere Antidiabetika, hypoglykämischen Wirkstoffe, HMGCOA-Reduktase Inhibitoren, Cholesterinresorptionsinhibitoren, PPAR gamma Agonisten, PPAR alpha Agonisten, PPAR alpha/gamma Agonisten, PPAR delta Agonisten, Fibrate, MTP-Inhibitoren, Gallensäureresorptionsinhibitoren, MTP-Inhibitoren, CETP-Inhibitoren, polymere Gallensäureadsorber, LDL-Rezeptorinducer, ACAT-Inhibitoren, Antioxidantien, Lipoprotein-Lipase Inhibitoren, ATP-Citrat-Lyase Inhibitoren, Squalen Synthetase Inhibitoren, Lipoprotein(a) Antagonisten, HM74A Rezeptor Agonisten, Lipase Inhibitoren, Insuline, Sulfonylharnstoffe, Biguanide, Meglitinide, Thiazolidindione, α-Glukosidase-Inhibitoren, auf den ATP-abhängigen Kaliumkanal der Betazellen wirkende Wirkstoffe, Glykogen Phosphorylase Inhibitoren, Glukagon-Rezeptor-Antagonisten, Aktivatoren der Glukokinase, Inhibitoren der Glukoneogenese, Inhibitoren der Fructose-1,6-biphosphatase, Modulatoren des Glukosetransporters-4, Inhibitoren der Glutamin-Fructose-6-Phosphat-Amidotransferase, Inhibitoren der Dipeptidylpeptidase-IV, Hemmstoffe der 11-beta-Hydroxysteroid-Dehydrogenase-1, Inhibitoren der Protein-Tyrosin-Phosphatase-1 B, Modulatoren des natrium-abhängigen Glukosetransporters 1 oder 2, Modulatoren des GPR40, Inhibitoren der hormon-sensitiven Lipase, Hemmstoffe der Acetyl-CoA Carboxylase, Inhibitoren der Phosphoenolpyruvatcarboxykinase, Inhibitoren der Glykogen Synthase Kinase-3 beta, Inhibitoren der Protein Kinase C beta, Endothelin-A-Rezeptor Antagonisten, Inhibitoren der I kappaB Kinase, Modulatoren des Glukocorticoidrezeptors, CART-Agonisten, NPY-Agonisten, MC4-Agonisten, Orexin-Agonisten, H3-Agonisten, TNF-Agonisten, CRF-Agonisten, CRF BP-Antagonisten, Urocortin-Agonisten, β3-Agonisten, CB1-Rezeptor Antagonisten ,MSH (Melanocyt-stimulierendes Hormon)-Agonisten, CCK-Agonisten, Serotonin-Wiederaufnahme-Inhibitoren, gemischte Sertonin- und noradrenerge Verbindungen, 5HT-Agonisten, Bombesin-Agonisten, Galanin-Antagonisten, Wachstumshormone, Wachstumshormon freisetzende Verbindungen, TRH-Agonisten, entkoppelnde Protein 2- oder 3-Modulatoren, Leptinagonisten, DA-Agonisten (Bromocriptin, Doprexin), Lipase/Amylase-Inhibitoren, PPAR-Modulatoren, RXR-Modulatoren oder TR-β-Agonisten oder Amphetamine enthält.

11. Arzneimittel, gemäß einem der Ansprüche 7, 8, 9 oder 10, **dadurch gekennzeichnet, daß** es als weiteren Hilfsstoff eine oder mehrere Metallsalze enthält.

12. Verbindungen der Formel I, gemäß einem oder mehreren der Ansprüche 1 bis 5 zur Anwendung in der Behandlung von Lipidstoffwechselstörungen.

13. Verfahren zur Herstellung eines Arzneimittels enthaltend die Verbindungen der Formel I, gemäß einem oder mehreren der Ansprüche 1 bis 5, **dadurch gekennzeichnet, daß** der Wirkstoff mit einem pharmazeutisch geeigneten Träger vermischt wird und diese Mischung in eine für die Verabreichung geeignete Form gebracht wird.

14. Verwendung der Verbindungen der Formel I, gemäß einem oder mehreren der Ansprüche 1 bis 5 zur Herstellung eines Medikaments zur Behandlung von Hyperlipidämie.

15. Verwendung der Verbindungen der Formel I, gemäß einem oder mehreren der Ansprüche 1 bis 5 zur Herstellung eines Medikaments zur Senkung des Serumcholesterinspiegels.

16. Verwendung der Verbindungen der Formel I, gemäß einem oder mehreren der Ansprüche 1 bis 5 zur Herstellung eines Medikaments zur Behandlung arteriosklerotischer Erscheinungen.

17. Verwendung der Verbindungen der Formel I, gemäß einem oder mehreren der Ansprüche 1 bis 5 zur Herstellung eines Medikaments zur Behandlung von Insulin Resistenz.

18. Verwendung der Verbindungen gemäß einem oder mehreren der Ansprüche 1 bis 5 zur Herstellung eines Medikamentes zur Behandlung von ZNS-Erkrankungen.

19. Verwendung der Verbindungen gemäß einem oder mehreren der Ansprüche 1 bis 5 zur Herstellung eines Medikamentes zur Behandlung von Schizophrenie.

20. Verwendung der Verbindungen gemäß einem oder mehreren der Ansprüche 1 bis 5 zur Herstellung eines Medikamentes zur Behandlung von Alzheimer.

## Claims

1. A compound of the formula I 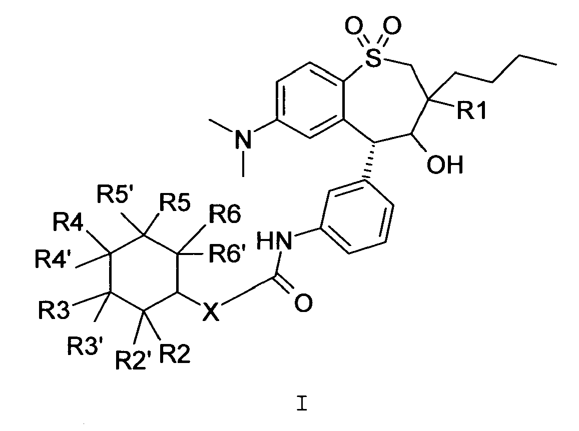 in which the meanings are
X CH₂, O, NH;
R1 (C₁-C₄)alkyl;
R2, R2', R3, R3', R4, R4', R5, R5', R6, R6' independently of one another H, OH, F, Cl, Br, I, CF₃, NO₂, N₃, CN, COOH, COO(C₁-C₆)alkyl, CONH₂, CONH(C₁-C₆)alkyl, CON[(C₁-C₆)alkyl]₂, (C₁-C₆)alkyl, (C₂-C₆)alkenyl, (C₂-C₆)alkynyl, O-(C₁-C₆)alkyl, where one, more than one, or all hydrogen(s) in the alkyl radicals may be replaced by fluorine; C(=NH)(NH₂), PO₃H₂, SF₅, SO₃H, OSO₃H, SO₂-NH₂, SO₂NH (C₁-C₆) alkyl, SO₂N[(C₁-C₆)alkyl]₂, S-(C₁-C₆)alkyl, S-(CH₂₎ₙ-phenyl, SO-(C₁-C₆)alkyl, SO-(CH₂)ₙ-phenyl, SO₂-(C₁-C₆)alkyl, SO₂-(CH₂)ₙ-phenyl, where n may be 0-6, and the phenyl radical may be substituted up to twice by F, Cl, Br, OH, CF₃, NO₂, CN, OCF₃, O-(C₁-C₆)alkyl, (C₁-C₆)alkyl, NH₂;
NH₂, NH-(C₁-C₆)alkyl, N((C₁-C₆)alkyl)₂, NH(C₁-C₇)acyl, phenyl, O-(CH₂)ₙ-phenyl, where n may be 0-6, where the phenyl ring may be substituted one to 3 times by F, Cl, Br, I, OH, CF₃, NO₂, CN, OCF₃, O-(C₁-C₆)alkyl, (C₁-C₆) alkyl, NH₂, NH(C₁-C₆)alkyl, N((C₁-C₆)alkyl)₂, SO₂-CH₃, COOH, COO-(C₁-C₆)alkyl, CONH₂;
and the pharmaceutically acceptable salts thereof.

2. A compound of the formula I as claimed in claim 1, wherein the meanings are
X O, NH;
R1 (C₁-C₄) alkyl;
R2, R2', R3, R3', R4, R4', R5, R5' , R6, R6' independently of one another H, OH, F, CI, Br, I, CF₃, NO₂, N₃, CN, COOH, COO(C₁-C₆)alkyl, CONH₂, CONH(C₁-C₆)alkyl, CON[(C₁-C₆)alkyl]₂, (C₁-C₆) alkyl, (C₂-C₆) alkenyl, (C₂-C₆)alkynyl, O-(C₁-C₆)alkyl, where one, more than one, or all hydrogen(s) in the alkyl radicals may be replaced by fluorine; C(=NH) (NH₂) , PO₃H₂, SF₅, SO₃H, OSO₃H, SO₂-NH₂, SO₂NH(C₁-C₆)alkyl, SO₂N[(C₁-C₆)alkyl]₂, S-(C₁-C₆)alkyl, S-(CH₂)ₙ-phenyl, SO-(C₁-C₆)alkyl, SO-(CH₂)ₙ-phenyl, SO₂-(C₁-C₆) alkyl, SO₂-(CH₂)ₙ-phenyl, where n may be 0-6, and the phenyl radical may be substituted up to twice by F, Cl, Br, OH, CF₃, NO₂, CN, OCF₃, O-(C₁-C₆)alkyl, (C₁-C₆) alkyl, NH₂;
NH₂, NH- (C₁-C₆) alkyl, N((C₁-C₆)alkyl)₂, NH (C₁-C₇) acyl, phenyl, O-(CH₂)ₙ-phenyl, where n may be 0-6, where the phenyl ring may be substituted one to 3 times by F, Cl, Br, I, OH, CF₃, NO₂, CN, OCF₃, O-(C₁-C₆)alkyl, (C₁-C₆)alkyl, NH₂, NH(C₁-C₆)-alkyl, N((C₁-C₆)alkyl)₂, SO₂-CH₃, COOH, COO-(C₁-C₆) alkyl, CONH₂;
and the pharmaceutically acceptable salts thereof.

3. A compound of the formula I as claimed in claim 1 or 2, wherein the meanings are
X NH;
R1 (C₁-C₄) alkyl;
R2, R2', R3, R3', R4, R4', R5, R5', R6, R6' independently of one another H, OH, F, Cl, Br, I, CF₃, NO₂, N₃, CN, COOH, COO(C₁-C₆)alkyl, CONH₂, CONH (C₁-C₆) alkyl, CON [ (C₁-C₆) alkyl] ₂, (C₁-C₆)-alkyl, (C₂-C₆)-alkenyl, (C₂-C₆)-alkynyl, O-(C₁-C₆)-alkyl, where one, more than one, or all hydrogen(s) in the alkyl radicals may be replaced by fluorine; C (=NH) (NH₂) , PO₃H₂, SF₅, SO₃H, OSO₃H, SO₂-NH₂, SO₂NH (C₁-C₆) alkyl, SO₂N[(C₁-C₆)alkyl]₂, S-(C₁-C₆)alkyl, S-(CH₂)ₙ-phenyl, SO-(C₁-C₆)alkyl, SO-(CH₂)ₙ-phenyl, SO₂-(C₁-C₆)alkyl, SO₂-(CH₂)ₙ-phenyl, where n may be 0-6, and the phenyl radical may be substituted up to twice by F, Cl, Br, OH, CF₃, NO₂, CN, OCF₃, O-(C₁-C₆)alkyl, (C₁-C₆)alkyl, NH₂; NH₂, NH-(C₁-C₆)alkyl, N((C₁-C₆)alkyl)₂, NH(C₁-C₇)acyl, phenyl, O-(CH₂)ₙ-phenyl, where n may be 0-6, where the phenyl ring may be substituted one to 3 times by F, Cl, Br, I, OH, CF₃, NO₂, CN, OCF₃, O-(C₁-C₆)alkyl, (C₁-C₆)alkyl, NH₂, NH(C₁-C₆)-alkyl, N((C₁-C₆)alkyl)₂, SO₂-CH₃, COOH, COO-(C₁-C₆)alkyl, CONH₂;
and the pharmaceutically acceptable salts thereof.

4. A compound of the formula I as claimed in one or more of claims 1 to 3, wherein the meanings are
X NH;
R1 (C₁-C₄)alkyl;
R2', R3', R4', R5', R6' H;
R2, R3, R4, R5, R6 independently of one another OH, F, Cl, Br, I, CF₃, NO₂, N₃, CN, COOH, COO(C₁-C₆)alkyl, CONH₂, CONH(C₁-C₆)alkyl, CON[(C₁-C₆)alkyl]₂, (C₁-C₆)alkyl, (C₂-C₆)-alkenyl, (C₂-C₆)alkynyl, O-(C₁-C₆)alkyl, where one, more than one, or all hydrogen(s) in the alkyl radicals may be replaced by fluorine; C (=NH) (NH₂) , PO₃H₂, SF₅, SO₃H, OSO₃H, SO₂-NH₂, SO₂NH(C₁-C₆) alkyl, SO₂N[(C₁-C₆)alkyl]₂, S-(C₁-C₆)alkyl, S-(CH₂)ₙ-phenyl, SO-(C₁-C₆)alkyl, SO-(CH₂)ₙ-phenyl, SO₂-(C₁-C₆)alkyl, SO₂-(CH₂)ₙ-phenyl, where n may be 0-6, and the phenyl radical may be substituted up to twice by F, Cl, Br, OH, CF₃, NO₂, CN, OCF₃, O-(C₁-C₆)alkyl, (C₁-C₆)alkyl, NH₂; NH₂, NH-(C₁-C₆)alkyl, N((C₁-C₆)alkyl)₂, NH(C₁-C₇)acyl, phenyl, O-(CH₂)ₙ-phenyl, where n may be 0-6, where the phenyl ring may be substituted one to 3 times by F, Cl, Br, I, OH, CF₃, NO₂, CN, OCF₃, O-(C₁-C₆)alkyl, (C₁-C₆) alkyl, NH₂, NH(C₁-C₆)-alkyl, N((C₁-C₆)alkyl)₂, SO₂-CH₃, COOH, COO-(C₁-C₆)alkyl, CONH₂;
and the pharmaceutically acceptable salts thereof.

5. A compound of the formula I as claimed in one or more of claims 1 to 3, wherein the meanings are
X NH;
R1 (C₁-C₄)alkyl;
R2', R3', R4', R5', R6' H;
R2, R3, R4, R5, R6 independently of one another OH, O-(C₁-C₆)alkyl, where one, more than one, or all hydrogen(s) in the alkyl radicals may be replaced by fluorine; OSO₃H, NH₂, NH-(C₁-C₆)alkyl, N((C₁-C₆)alkyl)₂, O-(CH₂)ₙ-phenyl, where n may be 0-6 and where the phenyl ring may be substituted one to 3 times by F, Cl, Br, I, OH, CF₃, NO₂, CN, OCF₃, O-(C₁-C₆)alkyl, (C₁-C₆)alkyl, NH₂, NH(C₁-C₆)alkyl, N((C₁-C₆)alkyl)₂, SO₂-CH₃, COOH, COO-(C₁-C₆) alkyl, CONH₂;
and the pharmaceutically acceptable salts thereof.

6. A compound of the formula I as claimed in one or more of claims 1 to 5, for use as pharmaceutical.

7. A pharmaceutical comprising compounds of the formula I as claimed in one or more of claims 1 to 5.

8. A pharmaceutical comprising compounds of the formula I as claimed in one or more of claims 1 to 5 and at least one further active ingredient.

9. The pharmaceutical as claimed in claim 8, which comprises as further active ingredient one or more compounds which normalize lipid metabolism.

10. The pharmaceutical as claimed in claim 9, which comprises as further active ingredient one or more antidiabetics, hypoglycemic active ingredients, HMGCoA reductase inhibitors, cholesterol absorption inhibitors, PPAR gamma agonists, PPAR alpha agonists, PPAR alpha/gamma agonists, PPAR delta agonists, fibrates, MTP inhibitors, bile acid absorption inhibitors, MTP inhibitors, CETP inhibitors, polymeric bile acid adsorbents, LDL receptor inducers, ACAT inhibitors, antioxidants, lipoprotein lipase inhibitors, ATP-citrate lyase inhibitors, squalene synthetase inhibitors, lipoprotein(a) antagonists, HM74A receptor agonists, lipase inhibitors, insulins, sulfonylureas, biguanides, meglitinides, thiazolidinediones, α-glucosidase inhibitors, active ingredients which act on the ATP-dependent potassium channel of the beta cells, glycogen phosphorylase inhibitors, glucagon receptor antagonists, activators of glucokinase, inhibitors of gluconeogenesis, inhibitors of fructose-1,6-bisphosphatase, modulators of glucose transporter 4, inhibitors of glutamine-fructose-6-phosphate amidotransferase, inhibitors of dipeptidylpeptidase IV, inhibitors of 11-beta-hydroxysteroid dehydrogenase 1, inhibitors of protein tyrosine phosphatase 1B, modulators of the sodium-dependent glucose transporter 1 or 2, modulators of GPR40, inhibitors of hormone-sensitive lipase, inhibitors of acetyl-CoA carboxylase, inhibitors of phosphoenolpyruvate carboxykinase, inhibitors of glycogen synthase kinase-3 beta, inhibitors of protein kinase C beta, endothelin-A receptor antagonists, inhibitors of I kappaB kinase, modulators of the glucocorticoid receptor, CART agonists, NPY agonists, MC4 agonists, orexin agonists, H3 agonists, TNF agonists, CRF agonists, CRF BP antagonists, urocortin agonists, β3 agonists, CB1 receptor antagonists, MSH (melanocyte-stimulating hormone) agonists, CCK agonists, serotonin reuptake inhibitors, mixed serotoninergic and noradrenergic compounds, 5HT agonists, bombesin agonists, galanin antagonists, growth hormones, growth hormone-releasing compounds, TRH agonists, uncoupling protein 2 or 3 modulators, leptin agonists, DA agonists (bromocriptine, Doprexin), lipase/amylase inhibitors, PPAR modulators, RXR modulators or TR-β agonists or amphetamines.

11. The pharmaceutical as claimed in any of claims 7, 8, 9 or 10, which comprises as further excipient one or more metal salts.

12. A compound of the formula I as claimed in one or more of claims 1 to 5 for use in the treatment of lipid metabolism disorders.

13. A process for manufacturing a pharmaceutical comprising the compounds of the formula I as claimed in one or more of claims 1 to 5, which comprises mixing the active ingredient with a pharmaceutically suitable carrier and converting this mixture into a form suitable for administration.

14. The use of the compounds of the formula I as claimed in one or more of claims 1 to 5 in the manufacture of a medicament for the treatment of hyperlipidemia.

15. The use of the compounds of the formula I as claimed in one or more of claims 1 to 5 in the manufacture of a medicament for lowering the serum cholesterol level.

16. The use of the compounds of the formula I as claimed in one or more of claims 1 to 5 in the manufacture of a medicament for the treatment of arteriosclerotic manifestations.

17. The use of the compounds of the formula I as claimed in one or more of claims 1 to 5 in the manufacture of a medicament for the treatment of insulin resistance.

18. The use of the compounds as claimed in one or more of claims 1 to 5 in the manufacture of a medicament for the treatment of CNS disorders.

19. The use of the compounds as claimed in one or more of claims 1 to 5 in the manufacture of a medicament for the treatment of schizophrenia.

20. The use of the compounds as claimed in one or more of claims 1 to 5 in the manufacture of a medicament for the treatment of Alzheimer's.

## Revendications

1. Composés de formule I, 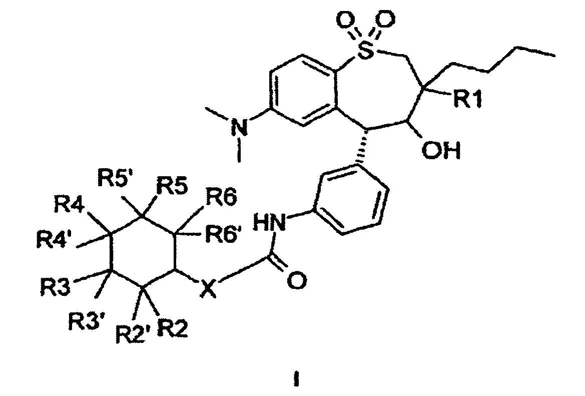 dans laquelle
X signifie CH₂, O, NH ;
R1 signifie (C₁-C₄)-alkyle ;
R2, R2', R3, R3', R4, R4', R5, R5', R6, R6' signifient, indépendamment l'un de l'autre H, OH, F, Cl, Br, I, CF₃, NO₂, N₃, CN, COOH, COO(C₁-C₆)-alkyle, CONH₂, CONH(C₁-C₆)-alkyle, CON[(C₁-C₆)-alkyle]₂, (C₁-C₆)-alkyle, (C₂-C₆)-alcényle, (C₂-C₆)-alcynyle, O-(C₁-C₆)-alkyle, où, dans les radicaux alkyle, un, plusieurs ou tous les hydrogène(s) peuvent être remplacés par fluor ; C(=NH) (NH₂), PO₃H₂, SF₅, SO₃H, OSO₃H, SO₂-NH₂, SO₂NH(C₁-C₆)-alkyle, SO₂N[(C₁-C₆)-alkyle]₂, S-(C₁-C₆)-alkyle, S-(CH₂)ₙ-phényle, SO-(C₁-C₆)-alkyle, SO-(CH₂)ₙ-phényle, SO₂-(C₁-C₆)-alkyle, SO₂-(CH₂)ₙ-phényle, où n peut être = 0-6 et le radical phényle peut être jusqu'à disubstitué par F, Cl, Br, OH, CF₃, NO₂, CN, OCF₃, O-(C₁-C₆)-alkyle, (C₁-C₆)-alkyle, NH₂ ; NH₂, NH-(C₁-C₆)-alkyle, N((C₁-C₆)-alkyle)₂, NH(C₁-C₇)-acyle, phényle, O-(CH₂)ₙ-phényle, où n peut être = 0-6, où le cycle phényle peut être monosubstitué à trisubstitué par F, Cl, Br, I, OH, CF₃, NO₂, CN, OCF₃, O-(C₁-C₆)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ;
ainsi que leurs sels pharmaceutiquement acceptables.

2. Composés de formule I, selon la revendication 1, **caractérisés en ce que**
X signifie O, NH ;
R1 signifie (C₁-C₄)-alkyle ;
R2, R2', R3, R3', R4, R4', R5, R5', R6, R6' signifient, indépendamment l'un de l'autre H, OH, F, Cl, Br, I, CF₃, NO₂, N₃, CN, COOH, COO(C₁-C₆)-alkyle, CONH₂, CONH(C₁-C₆)-alkyle, CON[(C₁-C₆)-alkyle]₂, (C₁-C₆)-alkyle, (C₂-C₆)-alcényle, (C₂-C₆)-alcynyle, O-(C₁-C₆)-alkyle, où, dans les radicaux alkyle, un, plusieurs ou tous les hydrogène(s) peuvent être remplacés par fluor ; C(=NH)(NH₂), PO₃H₂, SF₅, SO₃H, OSO₃H, SO₂-NH₂, SO₂NH(C₁-C₆)-alkyle, SO₂N[(C₁-C₆)-alkyle]₂, S-(C₁-C₆)-alkyle, S-(CH₂)ₙ-phényle, SO-(C₁-C₆)-alkyle, SO-(CH₂)ₙ₋phényle, SO₂-(C₁-C₆) -alkyle, SO₂-(CH₂)ₙ-phényle, où n peut être = 0-6 et le radical phényle peut être jusqu'à disubstitué par F, Cl, Br, OH, CF₃, NO₂, CN, OCF₃, O-(C₁-C₆)-alkyle, (C₁-C₆)-alkyle, NH₂ ; NH₂, NH-(C₁-C₆)-alkyle, N((C₁-C₆)-alkyle)₂, NH(C₁-C₇)-acyle, phényle, O-(CH₂)ₙ-phényle, où n peut être = 0-6, où le cycle phényle peut être monosubstitué à trisubstitué par F, Cl, Br, I, OH, CF₃, NO₂, CN, OCF₃, O-(C₁-C₆)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ;
ainsi que leurs sels pharmaceutiquement acceptables.

3. Composés de formule I, selon la revendication 1 ou 2, **caractérisés en ce que**
X signifie NH ;
R1 signifie (C₁-C₄)-alkyle ;
R2, R2', R3, R3', R4, R4', R5, R5', R6, R6' signifient, indépendamment l'un de l'autre H, OH, F, Cl, Br, I, CF₃, NO₂, N₃, CN, COOH, COO(C₁-C₆)-alkyle, CONH₂, CONH(C₁-C₆)-alkyle, CON[(C₁-C₆)-alkyle]₂, (C₁-C₆)-alkyle, (C₂-C₆)-alcényle, (C₂-C₆)-alcynyle, O-(C₁-C₆)-alkyle, où, dans les radicaux alkyle, un, plusieurs ou tous les hydrogène(s) peuvent être remplacés par fluor ; C(=NH)(NH₂), PO₃H₂, SF₅, SO₃H, OSO₃H, SO₂-NH₂, SO₂NH(C₁-C₆)-alkyle, SO₂N[(C₁-C₆)-alkyle]₂, S-(C₁-C₆)-alkyle, S-(CH₂)ₙ-phényle, SO-(C₁-C₆)-alkyle, SO-(CH₂)ₙ-phényle, SO₂-(C₁-C₆) -alkyle, SO₂-(CH₂)ₙ-phényle, où n peut être = 0-6 et le radical phényle peut être jusqu'à disubstitué par F, Cl, Br, OH, CF₃, NO₂, CN, OCF₃, O-(C₁-C₆)-alkyle, (C₁-C₆)-alkyle, NH₂ ; NH₂, NH-(C₁-C₆)-alkyle, N((C₁-C₆)-alkyle)₂, NH(C₁-C₇)-acyle, phényle, O-(CH₂)ₙ-phényle, où n peut être = 0-6, où le cycle phényle peut être monosubstitué à trisubstitué par F, Cl, Br, I, OH, CF₃, NO₂, CN, OCF₃, O-(C₁-C₆)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ;
ainsi que leurs sels pharmaceutiquement acceptables.

4. Composés de formule I selon l'une ou plusieurs des revendications 1 à 3, **caractérisés en ce que**
X signifie NH ;
R1 signifie (C₁-C₄)-alkyle ;
R2', R3', R4', R5', R6' signifient H ;
R2, R3, R4, R5, R6 signifient, indépendamment l'un de l'autre OH, F, Cl, Br, I, CF₃, NO₂, N₃, CN, COOH, COO(C₁-C₆)-alkyle, CONH₂, CONH(C₁-C₆)-alkyle, CON[(C₁-C₆)-alkyle]₂, (C₁-C₆)-alkyle, (C₂-C₆)-alcényle, (C₂-C₆)-alcynyle, O-(C₁-C₆)-alkyle, où, dans les radicaux alkyle, un, plusieurs ou tous les hydrogène(s) peuvent être remplacés par fluor ; C(=NH)(NH₂), PO₃H₂, SF₅, SO₃H, OSO₃H, SO₂-NH₂, SO₂NH(C₁-C₆)-alkyle, SO₂N[(C₁-C₆)-alkyle]₂, S-(C₁-C₆)-alkyle, S-(CH₂)ₙ-phényle, SO-(C₁-C₆)-alkyle, SO-(CH₂)ₙ-phényle, SO₂-(C₁-C₆)-alkyle, SO₂-(CH₂)ₙ-phényle, où n peut être = 0-6 et le radical phényle peut être jusqu'à disubstitué par F, Cl, Br, OH, CF₃, NO₂, CN, OCF₃, O-(C₁-C₆)-alkyle, (C₁-C₆)-alkyle, NH₂ ; NH₂, NH-(C₁-C₆)-alkyle, N((C₁-C₆)-alkyle)₂, NH(C₁-C₇)-acyle, phényle, O-(CH₂)ₙ-phényle, où n peut être = 0-6, où le cycle phényle peut être monosubstitué à trisubstitué par F, Cl, Br, I, OH, CF₃, NO₂, CN, OCF₃, O-(C₁-C₆)-alkyle, (C₁-C₆)-alkyle, NH₂, NH(C₁-C₆)-alkyle, N((C₁-C₆)-alkyle)₂, SO₂-CH₃, COOH, COO-(C₁-C₆)-alkyle, CONH₂ ;
ainsi que leurs sels pharmaceutiquement acceptables.

5. Composés de formule I selon l'une ou plusieurs des revendications 1 à 3, **caractérisés en ce que**
X signifie NH ;
R1 signifie (C₁-C₄)-alkyle ;
R2', R3', R4', R5', R6' signifient H ;
R2, R3, R4, R5, R6 signifient indépendamment l'un de l'autre OH, O-(C₁-C₆)-alkyle, où, dans les radicaux alkyle, un, plusieurs ou tous les hydrogène(s) peuvent être remplacés par fluor ; OSO₃H, NH₂, NH-(C₁-C₆)-alkyle, N((C₁-C₆)-alkyle)₂, O-(CH₂)ₙ-phényle, où n peut être = 0-6 et où le cycle phényle peut être monosubstitué à trisubstitué par F, Cl, Br, I, OH, CF₃, NO₂, CN, OCF₃, O-(C₁-C₆)-alkyle, (C₁-C₆)-alkyle, NH₂, NH(C₁-C₆)-alkyle, N ( (C₁-C₆) -alkyle) ₂, SO₂-CH₃, COOH, COO-(C₁-C₆)-alkyle, CONH₂ ;
ainsi que leurs sels pharmaceutiquement acceptables.

6. Composés de formule I selon l'une ou plusieurs des revendications 1 à 5 pour une utilisation comme médicament.

7. Médicament contenant des composés de formule I selon l'une ou plusieurs des revendications 1 à 5.

8. Médicament contenant des composés de formule I selon l'une ou plusieurs des revendications 1 à 5 et au moins une autre substance active.

9. Médicament selon la revendication 8, **caractérisé en ce qu'**il contient, comme autre substance active, un ou plusieurs composés qui normalisent le métabolisme des lipides.

10. Médicament selon la revendication 9, **caractérisé en ce qu'**il contient comme autre substance active un(e) ou plusieurs antidiabétiques, substances actives hypoglycémiques, inhibiteurs de HMGCoA-réductases, inhibiteurs de la résorption de cholestérol, agonistes de PPAR gamma, agonistes de PPAR alpha, agonistes de PPAR alpha/gamma, agonistes de PPAR delta, fibrates, inhibiteurs de MTP, inhibiteurs de la résorption de l'acide biliaire, inhibiteurs de MTP, inhibiteurs de CETP, adsorbants polymères de l'acide biliaire, inducteurs de récepteurs de LDL, inhibiteurs d'ACAT, antioxydants, inhibiteurs de la lipoprotéine-lipase, inhibiteurs de l'ATP-citrate, inhibiteurs de la squalène synthétase, antagonistes de la lipoprotéine (a), agonistes du récepteur HM74A, inhibiteurs de lipase, insulines, sulfonylurées, biguanides, méglitinides, thiazolidinediones, inhibiteurs d'α-glucosidase, substances actives agissant sur le canal potassique dépendant de l'ATP des cellules bêta, inhibiteurs de la glycogène phosphorylase, antagonistes du récepteur de glucagon, activateurs de la glucokinase, inhibiteurs de la gluconéogenèse, inhibiteurs de la fructose-1,6-biphosphatase, modulateurs du transporteur de glucose 4, inhibiteurs de la glutamine-fructose-6-phosphate-amidotransférase, inhibiteurs de la dipeptidylpeptidase-IV, inhibiteurs de la 11-bêta-hydroxystéroïde-déshydrogénase-1, inhibiteurs de la protéine-tyrosine-phosphatase-1B, modulateurs du transporteur de glucose 1 ou 2 dépendant du sodium, modulateurs du GPR40, inhibiteurs de la lipase sensible aux hormones, inhibiteurs de l'acétyl-CoA carboxylase, inhibiteurs de la phosphoénolpyruvate-carboxykinase, inhibiteurs de la glycogène synthase kinase-3 bêta, inhibiteurs de la protéine kinase C bêta, antagonistes du récepteur d'endothéline-A, inhibiteurs de la I kappaB kinase, modulateurs du récepteur des glucocorticoïdes, agonistes de CART, agonistes de NPY, agonistes de MC4, agonistes d'orexine, agonistes de H3, agonistes de TNF, agonistes de CRF, antagonistes de CRF BP, agonistes d'urocortine, agonistes de β3, antagonistes du récepteur de CB1, agonistes de la MSH (hormone stimulant les mélanocytes), agonistes de CCK, inhibiteurs de la résorption de la sérotonine, composés sérotoninergiques et noradrénergiques mixtes, agonistes de 5HT, agonistes de bombésine, antagonistes de galanine, hormones de croissance, composés libérant les hormones de croissance, agonistes de TRH, modulateurs 2 ou 3 de la protéine découplante, agonistes de la leptine, agonistes de DA (bromocriptine, doprexine), inhibiteurs de lipase/amylase, modulateurs de PPAR, modulateurs de RXR ou agonistes de TR-β ou amphétamines.

11. Médicament selon l'une quelconque des revendications 7, 8, 9 ou 10, **caractérisé en ce qu'**il contient comme autre adjuvant un ou plusieurs sels métalliques.

12. Composés de formule I selon l'une ou plusieurs des revendications 1 à 5 pour une utilisation dans le traitement de troubles du métabolisme des lipides.

13. Procédé pour la préparation d'un médicament contenant les composés de formule I selon l'une ou plusieurs des revendications 1 à 5, **caractérisé en ce que** la substance active est mélangée avec un support pharmaceutiquement approprié et ce mélange est amené dans une forme appropriée pour l'administration.

14. Utilisation des composés de formule I selon l'une ou plusieurs des revendications 1 à 5 pour la préparation d'un médicament destiné au traitement de l'hyperlipidémie.

15. Utilisation des composés de formule I selon l'une ou plusieurs des revendications 1 à 5 pour la préparation d'un médicament destiné à abaisser le niveau de cholestérol dans le sérum.

16. Utilisation des composés de formule I selon l'une ou plusieurs des revendications 1 à 5 pour la préparation d'un médicament destiné au traitement de phénomènes artérioscléreux.

17. Utilisation des composés de formule I selon l'une ou plusieurs des revendications 1 à 5 pour la préparation d'un médicament destiné au traitement de la résistance à l'insuline.

18. Utilisation des composés selon l'une ou plusieurs des revendications 1 à 5 pour la préparation d'un médicament destiné au traitement de maladies du système nerveux central.

19. Utilisation des composés selon l'une ou plusieurs des revendications 1 à 5 pour la préparation d'un médicament destiné au traitement de la schizophrénie.

20. Utilisation des composés selon l'une ou plusieurs des revendications 1 à 5 pour la préparation d'un médicament destiné au traitement de la maladie d'Alzheimer.
